# EUROPEAN PATENT APPLICATION

(11) **EP 1 813 941 A2**
(43) Date of publication of application: **01.08.2007**
(21) Application number: 06127299.3
(22) Date of filing: 28.12.2006
(51) Int. Cl.: G01N 33/487

(54) **Current damper for the study of cells**

(30) Priority: 28.12.2005 US 754195 P; 30.10.2006 US 855173 P
(71) Applicant: SENG ENTERPRISES LIMITED, Larnaca (CY)
(72) Inventor: Deutsch, Mordechai, 42 855 Doar-Na Lev-HaSharon (IL)
(74) Representative: Schmitz, Jean-Marie

(57) **Abstract**

A device for the study of cells including a vessel with a current damper including a damping component substantially disposed within the vessel is disclosed. The damping component reduces or eliminates currents formed by the addition of materials such as liquids to the vessel to prevent the movement of cells resting on the bottom surface of the vessel.

## Description

### FIELD AND BACKGROUND OF THE INVENTION

The present invention relates to the field of scientific devices and more particularly, to an improved device for the study of cells. Specifically, the present invention is of a device allowing the addition of a material to a vessel without disturbing cells held in the vessel.

Combinatorial methods in chemistry, cellular biology and biochemistry are essential for the near simultaneous preparation of multitudes of active entities such as molecules. Once such a multitude of molecules is prepared, it is necessary to study the effect of each one of the active entities on a living organism.

The study of the effects of stimuli such as exposure to active entities on living organisms is preferably initially performed on living cells. Since cell-functions include many interrelated pathways, cycles and chemical reactions, the study of an aggregate of cells, whether a homogenous or a heterogeneous aggregate, does not provide sufficiently detailed or interpretable results; rather a comprehensive study of the biological activity of an active entity may be advantageously performed by examining the effect of the active entity on a single isolated living cells. Thus, the use of single-cell assays is one of the most important tools for understanding biological systems and the influence thereupon of various stimuli such as exposure to active entities.

The combinatorial preparation of a multitudes of active entities coupled with the necessity of studying the effect of each one of the active entities on living organisms using single-cell assays, requires the development of high-throughput single live cell assays. There is a need for the study of real-time responses to stimuli in large and heterogeneous cell populations at an individual cell level. In such studies it is essential to have the ability to define multiple characteristics of each individual cell, as well as the individual cell response to the experimental stimulus of interest.

In the art, various methods and devices for studying living cells are known.

One method of studying cells involves placing cells on a bottom surface of a vessel and observing the behavior of the cell in response to stimuli. Typically used vessels include slides with recesses and Petri dishes. To allow for simultaneous study of distinct groups of cells exposed to similar or different stimuli, multiwell plates are most commonly used. Multiwell plates are substantially a group of individual vessels of a standard size physically associated in a standard way allowing for simplified simultaneous or sequential studies of different groups of cells. Multiwell plates having 6, 12, 24, 48, 96, 384 or even 1536 wells on a standard *ca.* 8.5 cm by *ca.* 12.5 cm footprint are well known in the art. Such multiwell plates are provided with an 2n by 3n array of rectangular packed wells, n being an integer. The diameter of the wells of a plate depends on the number of wells and is generally greater than about 250 micrometers (for a 1536 well plate). The volume of the wells depends on the number of wells and the depth thereof but generally is greater than 5 x 10⁻⁶ liter (for a 1536 well plate). The standardization of the multiwell plate format is a great advantage for researchers, allowing the use of standardized products including robotic handling devices, automated sample handlers, sample dispensers, plate readers, observation components, plate washers, software and such accessories as multifilters.

When a vessel having a planar bottom surface is used to study cells, the cells are most often studied as a group having an aggregate of properties of the individual cells. Since the cells are studied as a group, in such studies the identity of individual cells is not important. Such studies are of limited utility due to the fact that naturally occuring cell populations are rarely homogenous and often it is the heterogenity and the differences of behavior of cells that is interesting.

Efforts have been made to use vessels having a planar bottom surface to study cells as individuals but such efforts are plagued with many difficulties. A first difficulty is that cells have a tendency to clump together in variably sized groups at random locations, and often stack one on top of the other. The clumping and stacking of cells together makes it virtually impossible to delineate the borders of one cell from another, see discussion in unpublished PCT Patent Application No. IL2005/000719 of the inventor. It is thus virtually impossible to identify which cell has a given behavior. Further, the fact that cells are randomly distributed over a featureless surface makes it impossible to definitely differentiate one cell from another without continuous observation of the cell.

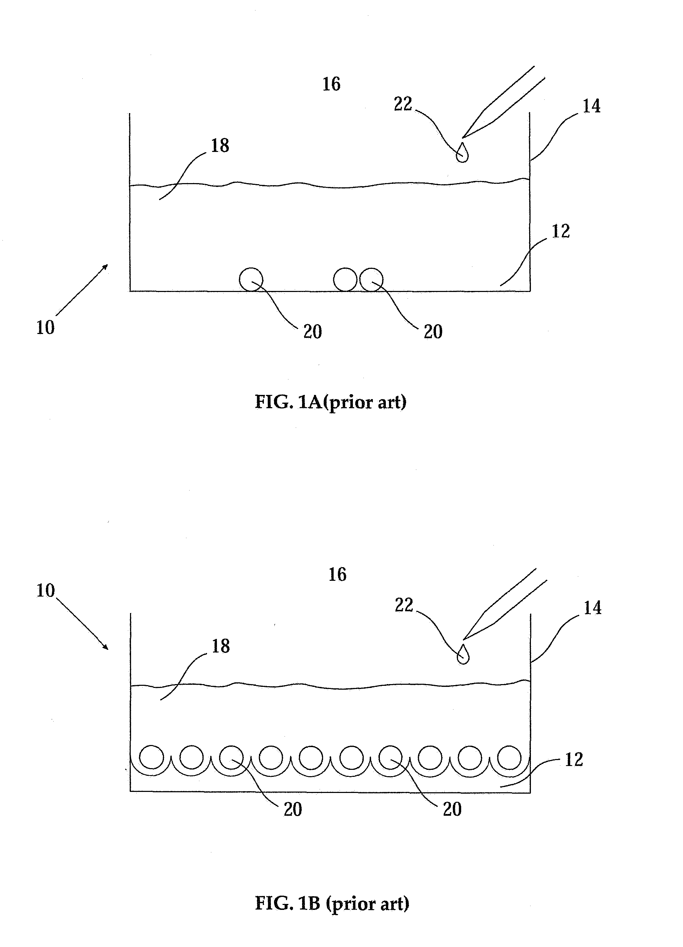

The greatest difficulty limiting the utility of such methods is that even the slightest current, whether caused by addition of a material to the vessel or by movement, e.g. incidental jostling, of the vessel causes the cells to move randomly leading to the loss of identity of the cells and rendering experiments difficult to perform, limited in scope and slow. In Figure 1A, a microwell 10 defined by a bottom surface 12 and vessel walls 14 is schematically depicted in cross section. A plurality of cells 20 rest at various locations on bottom surface 12. Cell density is relatively low to reduce as much as possible the clumping of cells 20. When a material 22 is added from surroundings 16, material 22 enters vessel 10 and mixes with liquid 18 held in vessel 10. When material 22 is added currents formed by the impact of material 22 with liquid 18 are sufficient to cause cells 20 to shift from a given location.

It is known to provided vessels having cell-localizing features arranged in arrays on a planar surface. Cells are held in a specific location that is individually addressable allowing the identity of a given cell to be retained even without continuous observation. Many such devices bind or adhere to the surface of the cells or deform the shape of the cells, adversely effecting the results of performed studies, see for example Mrksich and Whitesides, Ann. Rev. Biophys. Biomol. Struct. 1996, 25, 55-78; Craighead et al., J. Vac. Sci. Technol. 1982, 20, 316; Singhvi et al., Science 1994, 264, 696-698; Aplin and Hughes, Analyt. Biochem. 1981, 113, 144-148, U.S. Patent No. 4,729,949, U.S. Patent No. 5,324,591, U.S. Patent No. 6,103,479 and PCT Patent Application No. US99/04473 published as WO 99/45357.

In PCT patent applications IL2001/00992 published as WO2003/035824, IL2004/000571 published as WO2004/113492 and IL2004/000194 published as WO2004/077009, all of the inventor, are provided devices provided with a plurality of picowells for the study of cells. In such devices, individual cells are held unadhered and in a substantially natural state in individual adressable picowells. The term "picowell" is general and includes such features as dimples, depressions, tubes and enclosures. Since cells range in size from about 1 micrometers to about 100 (or even more) micrometers diameter there is no single picowell size that is appropriate for holding a single cell of any type. That said, the dimensions of the typical individual picowell in the picowell-bearing components known in the art have dimensions of between about 1 micrometers up to about 200 micrometers, depending on the exact implementation. Using such devices large number of cells are studied as individuals. Complex experiments involving serial addition of reagents and the like are performed with dedicated microfluidics. Despite the unparalleled utility of the devices taught in PCT patent applications IL2001/00992 published as WO2003/035824, IL2004/000571 published as W02004/113492 and IL2004/000194 published as WO2004/077009, such devices have a number of disadvantages. A first disadvantage is the need for for flow generators and concomitant interfaces that increases the complexity of such devices. A second disadvantage is that the difficulties in the use of the device including loading, attaching flow generators and the like render the integration of such a device with a robotics system for automatised use impractical.

Problems associated with the need for flow generators and complex interfaces are overcome in unpublished PCT patent application IL2005/000801 of the inventor where a device including an array of picowells that is configured for easy coupling to a robotics system for automatised use is taught. Although extremely useful, the device taught in PCT patent application IL2005/000801 does not provide a general solution allowing the use of existing vessels for the study of cells.

In PCT patent application IL2004/000661 published as WO2005/007796 of the inventor is taught a well-bearing device, where on the bottom surface of each well is a plurality of picowells. A preferred embodiment of the device is substantially a standard 96-well plate where the bottom of each well is covered with a plurality of picowells. Such a device allows the use of standard robotics and other standard accessories to study cells, while the picowells allows a high density of cells to be held in a microwell without cell clumping and where each cell is adressable. Unfortunately under certain conditions currents in a liquid held in a well may cause cells held in picowells to move and thus lose identity. In Figure 1B a microwell 10 defined by a bottom surface 12 and vessel walls 14 of a device of PCT patent application IL2004/000661 is schematically depicted in cross section. As is seen, bottom surface 12 is entirely covered with picowells, each picowell configured to hold one cell 20 separated from other cells 20. When a material 22 is added from surroundings 16, material 22 enters vessel 10 and mixes with liquid 18 held in vessel 10. It has been found that when a relatively large amount of material 22 is added, or material 22 is added at a high velocity (for example when ejected from a pipette or an automatic injector such as used in robotic devices), currents formed by the impact of material 22 with liquid 18 may cause cells 20 to move from one picowell to another leading to the loss of the idenitity of cells 20.

It would be highly advantageous to have a device for the study of cells not having at least some of the disadvantages of the prior art.

### SUMMARY OF THE INVENTION

The present invention successfully addresses at least some of the shortcomings of the prior art by providing a damping component disposed within a cell-holding vessel, such as a microwell. When material (*e.g.*, a fluid or solid) is added to the vessel or when a device (*e.g.*, a probe or a detector) is placed in the vessel the damping component damps, that is to say, prevents or reduces currents, turbulence or flows that otherwise would cause cells held in the vessel, and especially resting on the bottom of the vessel, to move.

Thus, according to the teachings of the present invention there is provided a device for the study of cells comprising: (a) a vessel including a bottom surface and a wall having a bottom edge, a top edge defining a rim of the vessel, the rim surrounding an opening; and (b) a current damper including a damping component substantially disposed within the vessel.

In embodiments of the present invention, the damping component substantially divides the vessel into at least two volumes, a first volume including at least part of the bottom surface and a second volume in fluid communication with the surroundings and with the first volume.

In embodiments of the present invention the current damper is fixedly associated with the vessel. In embodiments of the present invention the current damper is discrete from the vessel. In embodiments of the present invention the damping component is discrete from the vessel.

In embodiments of the present invention the damping component comprises a damping surface. In embodiments of the present invention the damping surface is planar and is preferably substantially parallel to the bottom surface. In embodiments of the present invention the damping surface is not-planar, (*e.g.*, curved) or not parallel to the bottom surface.

In embodiments of the present invention the damping surface is porous. In embodiments of the present invention the damping surface is permeable to liquids. In embodiments of the present invention the damping surface is a net (that is, having a net-like shape or form) having relatively large spaces for fluids to pass therethrough. In embodiments of the present invention the damping surface is a porous membrane, especially a microporous membrane.

In embodiments of the present invention where the damping component substantially divides the vessel into two volumes, a first volume including at least part of the bottom surface and a second volume in fluid communication with the surroundings and with the first volume, preferably fluid communication between the surroundings and the first volume is substantially only via the second volume. In embodiments of the present invention the fluid communication between the first volume and the second volume is substantially only through a damping surface.

In embodiments of the present invention the damping component is configured to optionally allow substantially unimpeded fluid communication between the first volume and the surroundings. In embodiments of the present invention the substantially unimpeded fluid communication is through a gap in the damping surface. In embodiments of the present invention the substantially unimpeded fluid communication is past a side of the damping surface.

In embodiments of the present invention the current damper is configured to allow at least part of the damping component to rest on the surface of a liquid contained in the vessel. In embodiments of the present invention the damping component is configured to float on the surface of a liquid (especially an aqueous liquid) contained in the vessel.

In embodiments of the present invention the damping surface is configured to be entirely submerged in a liquid held in the vessel. In such an embodiment a current damper is more effective in also damping currents cause by movement of the vessel.

In embodiments of the present invention the vessel is substantially parallel-walled and the damping component has an outer edge having dimensions substantially similar to dimensions of a cross-section defined by the parallel walls.

In embodiments of the present invention the damping component is configured to maintain the damping surface at a substantially fixed distance above the bottom surface of the vessel. In embodiments of the present invention the fixed distance is no more than about 5 mm, no more than about 3 mm, no more than about 1.5 mm, no more than about 1 mm, no more than about 500 micrometers, no more than about 300 micrometers, no more than about 200 micrometers, no more than about 100 micrometers and even no more than about 50 micrometers

In embodiments of the present invention the current damper further comprises a retaining component configured to physically engage the top edge of the vessel. In embodiments of the present invention the retaining component and the damping component are fixedly associated. In embodiments of the present invention the retaining component and the damping component are moveably associated.

In embodiments of the present invention the current damper further comprises a retaining component configured to rest on the bottom surface of the vessel.

In embodiments of the present invention the retaining component and the damping component are fixedly associated. In embodiments of the present invention the retaining component and the damping component are reversibly associated.

In embodiments of the present invention the current damper comprises a plurality of damping components, each damping component configured to be disposed with a separate vessel. In embodiments of the present invention the plurality is selected from the group consisting of 6, 12, 24, 48, 96, 384 and 1536.

In embodiments of the present invention, the damping component comprises a permeable layer of small particles (loose or mutually fixedly attached) including voids therebeween defining a plurality of non-linear fluid channels through the layer. In embodiments, substantially all fluid paths through the layer are non-linear fluid channels. In embodiments, the damping component substantially divides the vessel into two volumes, a first volume including at least part of the bottom surface of the vessel and a second volume in fluid communication with the surroundings and with the first volume through the layer of small particles. In embodiments, fluid communication between the surroundings and the first volume is substantially only via the second volume and through the fluid channels of the layer of small particles. In embodiments, the damping component is configured to optionally allow substantially unimpeded fluid communication between the first volume and the surroundings, that is to say, not through the fluid channels of the layer of small particles.

Thus, according to the teachings of the present invention there is also provided a current damper, comprising: a) a layer including an upper surface, a lower surface and a width comprising a plurality of small particles (loose or mutually fixedly attached, e.g., by compression or sintering) including voids therebeween so as to define a plurality of non-linear fluid channels from the upper surface through the layer to the lower surface, rendering the layer permeable to fluids; and b) a component limiting the width of the layer to a size configured to fit inside a vessel for the study of cells. In embodiments, substantially all fluid channels through the layer are non-linear fluid channels.

In embodiments, the layer of particles is no less than about 0.5 mm thick, no less than about 1 mm thick, no less than about 2 mm thick and even no less than about 3 mm thick.

In embodiments, the width limiting component has dimensions so as to slidingly fit inside a vessel for the study of cells. In embodiments, the vessel for the study of cells are the individual wells of standard 6-, 12-, 24-, 48-, 96-, 384- or 1536- well plates with which one skilled in the art is acquainted.

In embodiments, the small particles comprising the layer are substantially homogenous in size. In embodiments, the small particles comprising the layer are substantially heterogeneous in size. In embodiments, the small particles comprising the layer are of substantially the same order of magnitude in size (largest particles are up to three times the volume of the smallest particles).

In embodiments, the layer comprises at least two sublayers, a first sublayer comprising small particles of a first average size and a second sublayer comprising small particles of a second average size different from the first average size.

In embodiments, the layer comprises small particles having a volume of no more than about 14x10⁶ micrometer³ (substantially equivalent to spheres having a 150 micrometer radius), no more than about 5.2x10⁵ micrometer³ (substantially equivalent to spheres having a 50 micrometer radius), no more than about 6.5x10⁴ micrometer³ (substantially equivalent to spheres having a 25 micrometer radius) and even no more than about 5.2x10² micrometer³ (substantially equivalent to spheres having a 10 micrometer radius).

In embodiments, the layer comprises small particles having a volume of no less than about 5.2x10⁻¹ micrometer³ (substantially equivalent to spheres having a 0.5 micrometer radius) and even no less than about 65 micrometer³ (substantially equivalent to spheres having a 2.5 micrometer radius).

In embodiments, the width limiting component comprises a wall, e.g., a circular wall.

In embodiments, the width limiting component is a wall of the vessel for the study of cells. In embodiments, the layer is fixedly attached to the vessel for the study of cells.

In embodiments, the width limiting component comprises a plurality of mutually fixedly attached particles, for example, the particles of the outer sides are fixedly attached for example by an adhesive, by compression or by sintering.

In embodiments, the lower surface of the layer of particles is substantially defined by a porous bottom retaining layer, for example a non particulate layer such as a porous membrane, a net, a mesh or a layer of mutually fixedly attached particles, e.g., a sintered layer of particles.

In embodiments, the upper surface of the layer of particles is substantially defined by a porous bottom retaining layer, for example a non particulate layer such as a porous membrane, a net, a mesh or a layer of mutually fixedly attached particles, e.g., a sintered layer of particles.

As noted above, in embodiments, the current damper is configured so when inside a vessel for the study of cells, the layer is no more than about 5 mm above the bottom surface of the vessel, no more than about 3 mm, no more than about 1.5 mm, no more than about 1 mm, no more than about 500 micrometers, no more than about 300 micrometers, no more than about 200 micrometers, no more than about 100 micrometers and even no more than about 50 micrometers above the bottom surface of the vessel.

In embodiments, the current damper comprises a retaining component configured to rest on the bottom surface of a vessel for the study of cells, for example a part of the width limiting component, for example legs attached to the bottom of a circular wall.

In embodiments, the current damper comprises a component to suspend the layer above the bottom surface of a vessel for the study of cells, for example a part of the width limiting component, for example a flange or a lip at the upper part of a circular wall.

In embodiments, the lower surface of the layer is configured to rest on the bottom surface of the vessel for the study of cells. In embodiments, the lower surface comprises particles such that when resting on a plane such as the bottom surface of a vessel for the study of cells, voids between the particles are sufficiently large for the accommodation of cells. In embodiments, the lower surface comprises particles having a volume of no less than about 6.5x10⁴ micrometer³ (equivalent to spheres having a 25 micrometer radius), no less than about 5.2x10⁵ micrometer³ (equivalent to spheres having a 50 micrometer radius) and even no less than about 14x10⁶ micrometer³ (equivalent to spheres having a 150 micrometer radius).

In embodiments, a current damper further comprises a bypass channel through the layer, defining a linear flow channel through the layer.

According to the teachings of the present invention there is provided a method for the study of cells comprising (a) providing a vessel including a bottom surface and a wall having a bottom edge, a top edge defining a rim of the vessel, the rim surrounding an opening, (b) placing at least one cell in a liquid (*i.e.,* a cell solution) in the vessel at a location on the bottom surface, (c) disposing a damping component of a current damper within the vessel, and (d) adding a material (*e.g.*, a liquid or a solid) to the vessel wherein the damping component damps currents thereby reducing movement of the at least one cell from the location as a result of the adding of the material.

In embodiments of the present invention, the damping component substantially divides the vessel into at least two volumes, a first volume including at least part of the bottom surface and a second volume in fluid communication with the surroundings and with the first volume. In embodiments of the present invention, the at least one cell is placed in the first volume. In embodiments of the present invention, adding the material is into the second volume. In embodiments, subsequent to the adding of the material into the second volume, the material passes into the first volume from the second volume.

In embodiments of the present invention the cell solution is placed on the bottom surface before the damping component is disposed within the vessel. In embodiments of the present invention the at least one cell is placed in the vessel on the bottom surface after the damping component is disposed within the vessel.

Disposing the damping component in the vessel includes during manufacture, for example integrally forming the damping component with the vessel or attaching during manufacture, and also includes laying a discrete damping component inside of the well before or after the cell solution is added to the vessel.

In embodiments of the present invention, the damping component comprises a damping surface and adding the material includes contacting the material with the damping surface.

In embodiments of the present invention, the damping surface is not planar.

In embodiments of the present invention, the damping surface is substantially unparallel to the bottom surface. In embodiments of the present invention, the damping surface is substantially perpendicular to the bottom surface.

In embodiments of the present invention, the damping surface is substantially entirely submerged in the liquid. The advantage of such an embodiment is that the damping component is then more effective at damping currents caused by movement of the vessel.

In embodiments of the present invention the damping surface is substantially planar. In embodiments of the present invention the damping surface is substantially parallel to the bottom surface of the vessel. In embodiments of the present invention the damping surface is substantially at a fixed distance from the bottom surface. In embodiments of the present invention the fixed distance is no more than about 5 mm, no more than about 3 mm, no more than about 1.5 mm, no more than about 1 mm, no more than about 500 micrometers, no more than about 300 micrometers, no more than about 200 micrometers, no more than about 100 micrometers and even no more than about 50 micrometers.

In embodiments of the present invention, the damping surface is porous and/or permeable and subsequent to adding the material, the material passes through the damping surface.

In embodiments of the present invention, at least part of the damping component (*e.g.,* the damping surface) rests (*e.g.,* floats) on the surface of the liquid in the well.

In embodiments of the present invention at least part of the damping component rests on the bottom surface of the vessel.

In embodiments of the present invention the damping component is suspended within the vessel above the bottom surface.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1A (prior art) schematically depicts addition of a material to a microwell of a 96-well plate;
FIG. 1B (prior art) schematically depicts addition of a material to a microwell of a 96-well plate provided with picowells as taught in PCT patent application No. IL2004/000661 published as WO2005/007796;
FIG. 2A depicts an embodiment of a current damper of the present invention;
FIG. 2B schematically depicts addition of a material to a microwell in which the current damper depicted in Figure 2A is disposed;
FIG. 2C schematically depicts removal of a liquid from a microwell in which the current damper depicted in Figure 2A is disposed;
FIG. 3 depicts an alternative embodiment of a current damper of the present invention;
FIG. 4 depicts an alternative embodiment of a current damper of the present invention;
FIGS. 5A and 5B depict an alternative embodiment of a current damper of the present invention;
FIG. 6 depicts an alternative embodiment of a current damper of the present invention;
FIG. 7 depicts an alternative embodiment of a current damper of the present invention;
FIG. 8 depicts an alternative embodiment of a current damper of the present invention;
FIG. 9 depicts an alternative embodiment of a current damper of the present invention;
FIG. 10 depicts an alternative embodiment of a current damper of the present invention;
FIGS. 11A and 11B depict an alternative embodiment of a current damper of the present invention;
FIG. 12 depicts an alternative embodiment of a current damper of the present invention;
FIG. 13 depicts an alternative embodiment of a current damper of the present invention;
FIG. 14 depicts an alternative embodiment of a current damper of the present invention;
FIG. 15 depicts an alternative embodiment of a current damper of the present invention including a bundle of microtubes;
FIG. 16A depicts an alternative embodiment of a current damper of the present invention including a spiral wall;
FIG. 16B depicts an alternative embodiment of a current damper of the present invention including rows of triangular baffles;
FIG. 17 depicts an alternative embodiment of a current damper of the present invention including planar baffles;
FIGS. 18A and 18B depict an alternative embodiment of a current damper of the present invention including a plurality of small particles;
FIG. 19 depicts an alternative embodiment of a current damper of the present invention including a two distinct layers, each comprising a plurality of different sized small particles; and
FIGS. 20A and 20B depict an alternative embodiment of a current damper of the present invention including a fluorinated polymer porous membrane as a damping component.

In the figures herein features such as cells 20 are depicted out of scale for illustrative purposes.

### EMBODIMENTS OF THE INVENTION

The present invention is of a device for the study of cells comprising a vessel for holding cells, where the cells rest on the bottom surface of the vessel, and a current damper including a damping component substantially disposed within the vessel. The present invention is also of a current damper configured to be placed in, or disposed within, a vessel. The present invention is also of a method for using a device of the present invention. While a cell or cells are resting on the bottom surface of the vessel a material (*e.g.*, a liquid or solid reagent) is added to the vessel or a device (*e.g.,* a probe or a detector) is placed in the vessel. The damping component preferably damps currents caused by the addition of the material or the placement of the device that would otherwise cause movement of the cell or cells. The damping component preferably also damps currents caused by movement of the vessel that would otherwise cause movement of the cell or cells.

The principles and uses of the teachings of the present invention may be better understood with reference to the accompanying description, figures and example. In the figures, like reference numerals refer to like parts throughout.

Before explaining at least one embodiment of the invention in detail, it is to be understood that the invention is not limited in its application to the details set forth herein. The invention can be implemented with other embodiments and can be practiced or carried out in various ways. It is also understood that the phraseology and terminology employed herein is for descriptive purpose and should not be regarded as limiting.

Generally, the nomenclature used herein and the laboratory procedures utilized in the present invention include techniques from the fields of biology, chemistry, engineering and physics. Such techniques are thoroughly explained in the literature.

Unless otherwise defined, all technical and scientific terms used herein have the same meaning as commonly understood by one of ordinary skill in the art to which the invention belongs. In addition, the descriptions, materials, methods, and examples are illustrative only and not intended to be limiting. Methods and materials similar or equivalent to those described herein can be used in the practice or testing of the present invention.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of".

The phrase "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts. Implementation of the methods of the present invention involves performing or completing selected tasks or steps manually, automatically, or a combination thereof.

Herein, the term "active entity" is understood to include chemical, biological or pharmaceutical entities including any natural or synthetic chemical or biological substance that influences a cell with which the entity is in contact. Typical active entities include but are not limited to active pharmaceutical ingredients, antibodies, antigens, biological materials, chemical materials, chromatogenic compounds, drugs, enzymes, fluorescent probes, immunogenes, indicators, ligands, nucleic acids, nutrients, peptides, physiological media, proteins, receptors, selective toxins and toxins.

Herein, by "indicator" is meant any active entity that upon interaction with some stimulus produces an observable effect. In the context of the present invention, by stimulus is meant, for example, a specific second active entity (such as a molecule) released by a cell and by observable effect is meant, for example, a visible effect, for example a change in color or emission of light.

Herein, by "picowell array" is meant a group of one or more picowells, preferably a plurality of picowells, preferably a plurality of picowells arranged in an orderly fashion.

Some embodiments of the present invention include components that are transparent or are made of a transparent material. By "transparent" is meant that the component or material is substantially transparent to at least one wavelength of light (preferably a range of wavelengths) in at least part of the visible light spectrum, the ultraviolet light spectrum and/or of infrared radiation, preferably the visible light spectrum.

Generally, the present invention is based on disposing a damping component in a vessel holding a liquid and having a bottom surface on which small particles such as a cell or cells rest. Generally, the damping component divides the vessel into at least two volumes. The first volume includes at least part of the bottom surface and therefore the cells and at least some liquid while the second volume is in fluid communication with the first volume through the damping component.

When a material is added directly into the first volume, for example by using a pipette, the speed and mass of the added material produces currents in the liquids that causes the cell or cells to move, causing a loss of identity of the cells as discussed in the introduction.

When material is added into the second volume currents are produced in the second volume. However the currents are damped by the current damper and do not substantially pass into the first volume. Rather, the added material passes through the damping component to contact the cells with little, no or substantially no disturbing of the cells.

Thus, the teachings of the present invention allows the addition of a material such as a liquid, reagent or active entity to a vessel where cells are resting on the bottom surface of the vessel without fear that the addition of the the material will cause currents that will move the cells.

In embodiments of the present invention, addition of materials to the first (cell-containing) volume from the surroundings is substantially only through the damping component via the second volume.

In embodiments of the present invention, the device is configured to allow optional bypassing of the damping component to allow addition of material to the first volume from the surroundings.

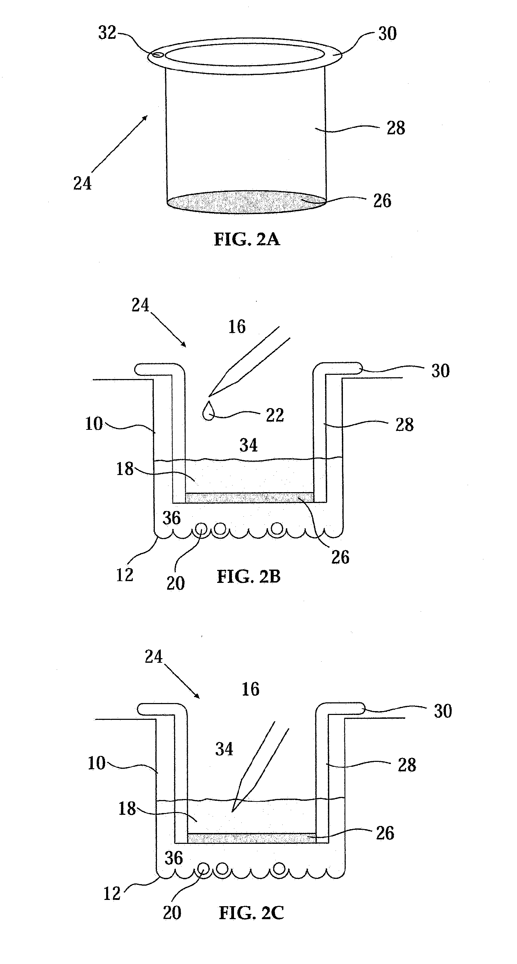

In Figure 2A, a current damper 24 of the present invention is depicted in perspective. Current damper 24 includes a damping surface 26, a circular wall 28 and a flange 30 provided with a pressure equalizing notch 32.

Damping surface 26 is a substantially planar circular piece of 3 micrometer microporous polycarbonate filter membrane attached to the bottom rim of circular wall 28. Although damping surface 26 depicted in Figure 2A is a microporous membrane, non-depicted embodiments of permeable damping surfaces of the present invention exist, for example where a damping surface is substantially a net or net-like arrangement of fibers or the like or an otherwise permeable membrane.

Circular wall 28 is configured to be disposed within an appropriate vessel, for example a microwell 10 of a standard 96-well plate or of a 96-well plate provided with picowells as described in PCT patent application IL2004/000661 published as WO2005/007796 of the Applicant, as depicted in Figures 2B and 2C.

Flange 30 is an integrally molded part of circular wall 28, flaring from the top rim of circular wall 28. Flange 30 constitutes a retaining component of current damper 24 and is configured to engage the top surface in the proximity of the rim of a microwell 10 to suspend damping surface 26 substantially in parallel to and at a distance of 200 micrometers above bottom surface 12. Generally the preferred distance that a damping surface is suspended above a bottom surface of a vessel is dependent on many factors such as vessel size or vessel volume and is determined for a specific implementation. That said, in embodiments of the present invention a damping surface is suspended no more than about 5 mm, no more than about 3 mm, no more than about 1.5 mm, no more than about 1 mm, no more than about 500 micrometers, no more than about 300 micrometers, no more than about 200 micrometers, no more than about 100 micrometers and even no more than about 50 micrometers above the bottom surface of a respective vessel.

The use of current damper 24 is schematically depicted in Figures 2B and 2C.

A physiological solution 18 including cells 20 is placed inside microwell 10. Cells 20 are allowed to settle into picowells on bottom surface 12. The damping component of current damper 24 consisting of circular wall 28 and damping surface 26 is placed inside microwell 10 so that flange 30 (constituting the retaining component of current damper 24) engages the top surface in the proximity of the rim of a microwell 10 as discussed above. As current damper 24 settles into microwell 10, trapped air escapes through pressure equalizing notch 32.

As is seen in Figures 2B and 2C, when current damper 24 is in place, vessel 10 is divided into two volumes: a second volume 34 substantially above damping surface 26 and a first volume 36 substantially between damping surface 26 and bottom surface 12. First volume 36 is in fluid communication with surroundings 16 substantially only through damping surface 26 via second volume 34. Damping surface 26 is entirely submerged in liquid 18 held in vessel 10. A substantially submerged or an entirely submerged damping surface such as 26 depicted in Figures 2B and 2C is more effective than other configurations in damping of currents caused by movement of a respective vessel such as 10 and is thus preferred.

In Figure 2B, addition of a material 22 to microwell 10 provided with current damper 24 is depicted. Material 22 placed in second volume 34 makes contact with and passes through damping surface 26 into first volume 36 and particularly to bottom surface 12 and cells 20. The addition of material 22 to second volume 34 causes currents, but as passage of material 22 is through damping surface 26, currents caused by the addition of material 22 to microwell 10 are substantially isolated to second volume 34 and damped in first volume 36 so cells 20 are only a little, not or substantially not disturbed and stay substantially in location on bottom surface 12.

In Figure 2C, removal of liquid 18 from microwell 10 provided with current damper 24 is depicted. Liquid 18 is removed from second volume 34, lowering the level of liquid 18 in second volume 34. Subsequently, liquid 18 in first volume 36 passes up through damping surface 26 into second volume 34 to equalize the respective liquid level. As the passage of liquid 18 is through damping surface 26, currents caused by removal of liquid 18 from microwell 10 are substantially isolated to second volume 34 and damped in first volume 36 so cells 20 are only a little, not or substantially not disturbed and stay substantially in location on bottom surface 12. It is important to note that current damper 24 is fashioned to be sufficiently heavy or is attached to microwell 10 in order to avoid buoyancy-induced shifting when liquid 18 is removed from microwell 10.

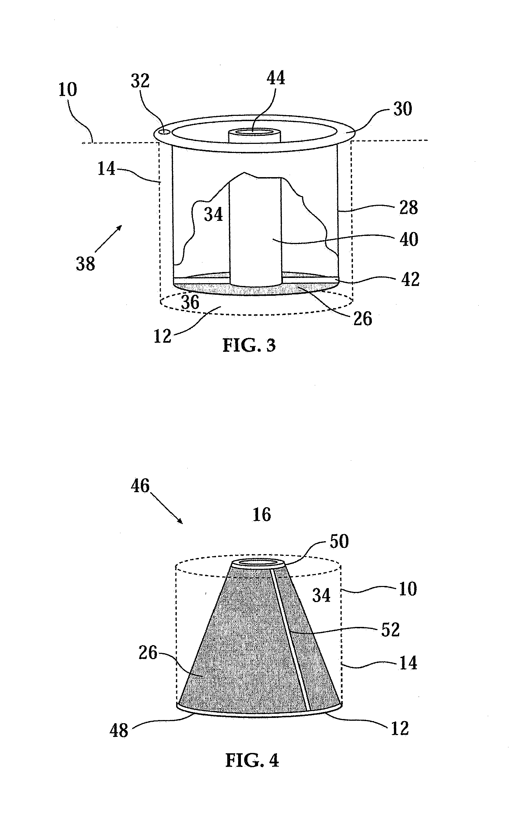

In Figure 3 is depicted a current damper 38 of the present invention in perspective with a portion cut out, disposed inside a vessel 10, depicted in phantom. Current damper 38 is similar to current damper 24 depicted in Figures 2A-2C with a significant difference that current damper 38 is provided with tubular internal wall 40 rigidly attached to circular wall 28 through ribs 42. As in current damper 24 depicted in Figures 2A-2C, current damper 38 is provided with a substantially planar porous damping surface 26.

When current damper 38 is disposed within a vessel 10, flange 30 engages the top surface in the proximity of the rim of vessel 10 so that damping surface 26 is suspended substantially in parallel to and at a fixed distance above bottom surface 12 of vessel 10. Further, when current damper 38 is in place, vessel 10 is divided into two volumes: a second toroidal volume 34 substantially above damping surface 26 and a first volume 36 substantially between damping surface 26 and bottom surface 12. First volume 36 is in fluid communication with surroundings 16 through damping surface 26 via second volume 34 and also directly and unimpeded through circular inner wall 40.

Damping component 38 is optionally used substantially as described above for damping component 24. The addition of a material 22 to second volume 34 causes currents, but as passage of material 22 is through damping surface 26, currents caused by the addition of material 22 to microwell 10 are substantially isolated to second volume 34 and damped in first volume 36 so cells 20 are only a little, not or substantially not disturbed and substantially stay in location on bottom surface 12. However, first volume 36 can also be accessed directly and unimpeded through upper opening 44 of circular inner wall 40. An advantage of direct and unimpeded access is that damping component 38 can be disposed in vessel 10 and subsequently a solution including cells 20 added through upper opening 44 directly into first volume 36 to settle onto bottom surface 12 of vessel 10.

In Figure 4 is depicted a current damper 46 of the present invention in perspective in place inside a vessel 10, depicted in phantom. Current damper 46 includes a lower ring 48 and an upper ring 50 conjoined by ribs 52 to define a truncated conical skeleton to which a non-planar microporous membrane damping surface 26 is attached.

For use, current damper 46 is placed inside an appropriate vessel 10 with lower ring 48, constituting a retaining component, resting on bottom surface 12 of vessel 10. Lower ring 48 is configured to contact vessel walls 14 of vessel 10 in proximity of bottom surface 12 to hold current damper 46 snugly in place. When current damper 46 is in place in vessel 10, vessel 10 is divided into two volumes: a second volume 34 substantially above damping surface 26 and a first volume substantially between damping surface 26 and bottom surface 12 of vessel 10. The first volume is in fluid communication with surroundings 16 through damping surface 26 via second volume 34 and also directly and unimpeded through the bore of upper ring 50.

Analogously to current damper 38 depicted in Figure 3, current damper 46 allows addition and removal of materials from the first volume either directly and unimpeded or through damping surface 26 to dampen potentially produced currents.

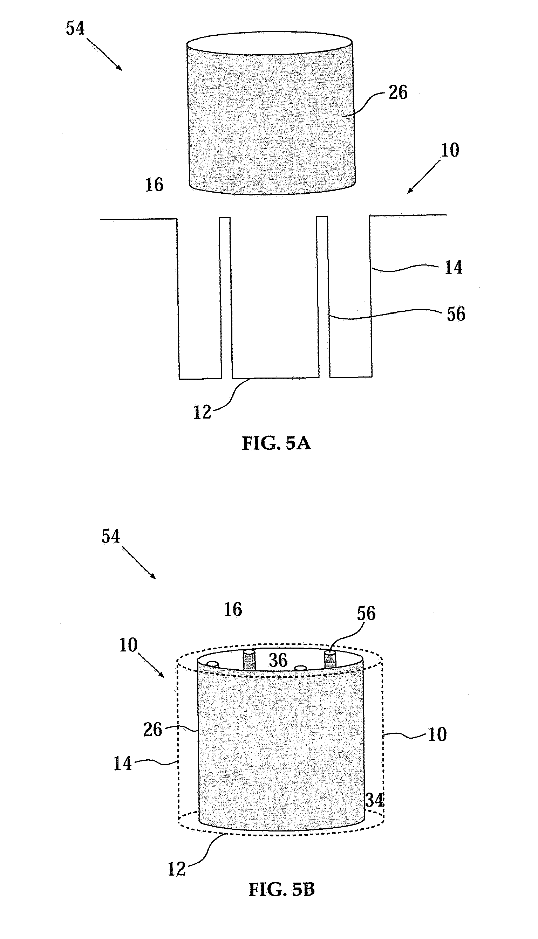

A current damper 54 of the present invention is depicted in a dissassembled state in Figure 5A (side view and perspective) and in Figure 5B in an assembled state (in perspective) as part of vessel 10 (depicted in phantom). Unlike the previously depicted embodiments of the device of the present invention, components of current damper 54 are fixedly associated with vessel 10. Current damper 54 includes a tubular microporous membrane damping surface 26 that is removeably held in place by support pegs 56 that are integrally formed with bottom surface 12 of vessel 10 and function as retaining components. Damping surface 26 is placed over support pegs 56 for assembly of current damper 54 so that vessel 10 is divided into two volumes: second volume 34 substantially the tubular volume between vessel walls 14 and damping surface 26 and first volume 36 substantially the cylindrical volume enclosed by damping surface 26.

For use, a solution containing cells is placed in first volume 36 so that the cells settle on bottom surface 12 inside first volume 36. Surroundings 16 are in fluid communication with first volume 36 both through damping surface 26 via second volume 34 or directly and unimpeded from the area of the opening of vessel 10 delineated by the top of damping surface 26. Analogously to current damper 38 depicted in Figure 3, current damper 54 allows addition and removal of materials from first volume 36 either directly and unimpeded or through damping surface 26 to dampen potentially produced currents.

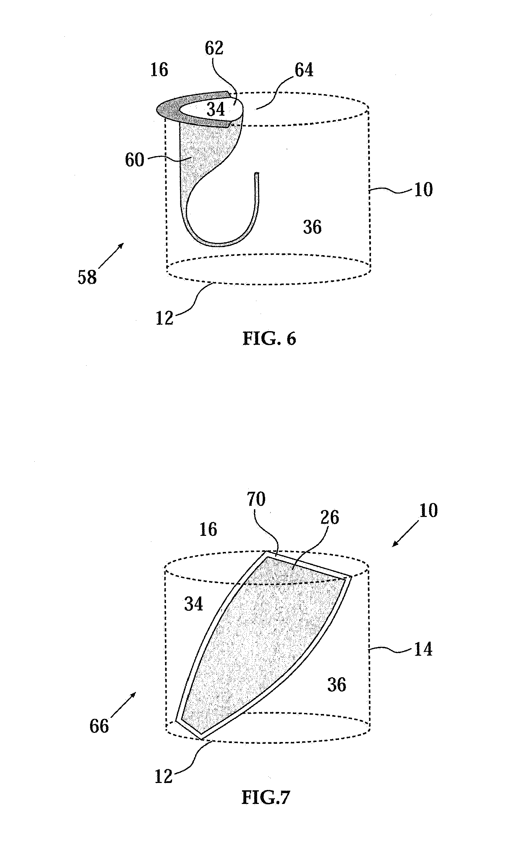

A current damper 58 of the present invention is depicted in Figure 6 disposed within vessel 10 depicted in phantom. Current damper 58 is fixedly associated with vessel 10 using adhesive. Unlike previously depicted embodiments of the present invention, current damper 58 does not include a damping surface. Current damper 58 is substantially a bent tube 60 having a relatively large diameter at an inlet 62 and a relatively small diameter at an outlet 64 substantially constituting a self-contained vessel and defining a second volume 34. The part of vessel 10 not taken up by current damper 58 constitutes a first volume 36. When a liquid material is introduced through inlet 62 into second volume 34, the material passes through bent tube 60 and emerges out into first volume 36 through outlet 64. The reduction of the diameter of bent tube 60 from inlet 62 to outlet 64 reduces the rate of entry of the material to first volume 36. Further, material exiting through outlet 64 flows along the outer wall of bent tube 60 in proximity of outlet 64. Both the reduced exit flow rate and the flow along the outer wall of bent tube 60 damps currents potentially produced by the addition of the material. For use, a solution containing cells is placed in first volume 36 so that the cells settle on bottom surface 12 inside first volume 36. Surroundings 16 are in fluid communication with first volume 36 both through second volume 34 via outlet 64 or directly and unimpeded through the top opening of vessel 10. Analogously to current damper 38 depicted in Figure 3, current damper 58 allows addition of materials to first volume 36 either directly and unimpeded or through second volume 34 to dampen potentially produced currents.

A current damper 66 of the present invention is depicted in Figure 7 disposed within vessel 10 depicted in phantom. Current damper 66 includes a frame 70 to which a planar microporous membrane damping surface 26 is attached. Frame 70 is fixedly attached by welding to bottom surface 12 and at two sides of vessel wall 14. In such a way, vessel 10 is divided into two volumes in fluid communication through damping surface 26: a second volume 34 substantially above damping surface 26 and a first volume 36 substantially between damping surface 26 and bottom surface 12 of vessel 10. First volume 36 is in fluid communication with surroundings 16 through damping surface 26 via second volume 34 and also directly and unimpeded through upper opening of vessel 10. Analogously to current damper 38 depicted in Figure 3, current damper 66 allows addition and removal of materials from first volume 36 either directly and unimpeded or through damping surface 26 to dampen potentially produced currents.

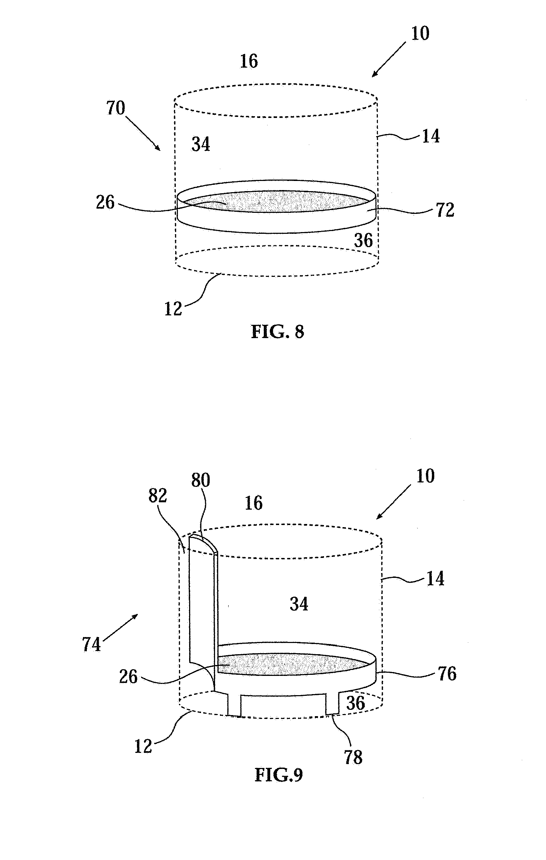

A current damper 70 of the present invention is depicted in Figure 8 disposed within vessel 10 depicted in phantom. Current damper 70 includes a circular frame 72 to which a planar microporous membrane damping surface 26 is attached. Current damper 70 is a discrete component configured to float on the surface of a liquid held in vessel 10 continuously maintaining damping surface 26 parallel to bottom surface 12 at a distance that varies with the depth of the liquid held in vessel 10. Circular frame 72 has an outer edge having dimensions substantially similar to those of a cross-section of vessel 10 so that circular frame 72 moves freely up and down inside vessel 10 with little space between circular frame 72 and vessel wall 14. In such a way, vessel 10 is divided into two volumes in fluid communication substantially exclusively through damping surface 26: a second volume 34 above damping surface 26 and a first volume 36 below damping surface 26. Surroundings 16 are in fluid communication with first volume 36 only through damping surface 26 via second volume 34. Analogously to current damper 24 depicted in Figures 2A-2C, current damper 70 damps currents potentially produced in first volume 36 when materials are added to second volume 34.

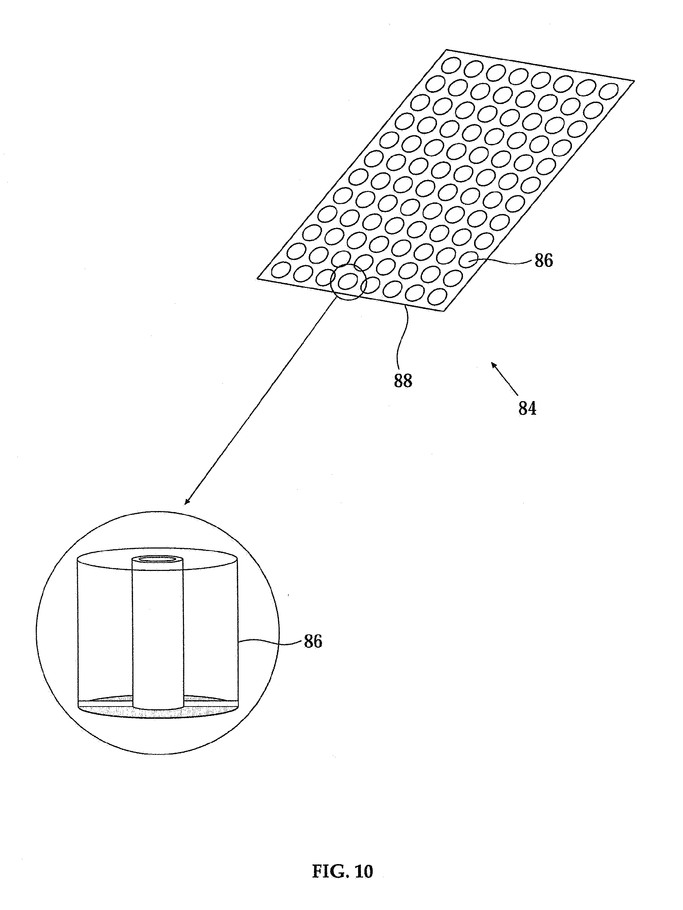

A current damper 74 of the present invention is depicted in Figure 9 disposed within vessel 10 depicted in phantom. Current damper 74 includes a frame 76 to which a planar microporous membrane damping surface 26 is attached. Current damper 74 is a discrete component configured to rest on bottom surface 12 of vessel 10 on integrally formed feet 78 which constitute retaining components of current damper 74. Feet 78 and frame 82 are configured to maintain damping surface 26 parallel with and at a fixed distance from bottom surface 12. Frame 76 is configured to snugly fit inside vessel 10 and to contact vessel walls 14 except in the vicinity of upwardly extending inwardly arcing solid wall 80. In such a way, vessel 10 is divided into two volumes: a second volume 34 above damping surface 26 and a first volume 36 below damping surface 26. Second volume 34 and first volume 36 are in fluid communication through damping surface 26. Further, as solid wall 80 arcs inwardly away from vessel wall 14, solid wall 80 defines a passage 82. Passage 82 allows direct and unimpeded fluid communication of first volume 36 with surroundings 16. Analogously to current damper 38 depicted in Figure 3, current damper 74 allows addition of materials to first volume 36 either directly and unimpeded through passage 82 past the side of damping surface 26 or through damping surface 26 to dampen potentially produced currents.

In Figure 10 is depicted a current damper 84 of the present invention. Current damper 84 is substantially planar and includes 96 individual damping components 86 arranged in an 8 by 12 array suitable for coupling with a standard 96 well plate. Each individual damping component 86 is substantially similar to current damper 38 depicted in Figure 3 with the exception that a plate 88 acts as a retaining component instead of a flange 30 of current damper 34 The size of each individual damping component 86 as well as the spacing and arrangement of the array is such that when current damper 84 is coupled with a standard 96 well plate, a damping component 86 is disposed inside each one of the 96 individual microwells. Whereas current damper 84 is provided with 96 individual damping components 86 to allow coupling with a standard 96 well plate, preferred non-depicted embodiments of the present invention are provided with 6, 12, 24, 48, 384 or 1536 individual damping components of the appropriate size and arranged in an array to allow coupling with standard format plates having 6, 12, 24, 48, 384 or 1536 microwells.

In embodiments of the present invention described above, a damping component of a current damper is substantially a porous membrane, especially a microporous membrane. For certain uses, such a damping component may be insufficiently effective. Often, a membrane must be wet to function efficiently what may be expensive (when the membrane is produced and prepackaged wet) or inconvenient (when the membrane is provided dry and must be immersed in a wetting solution for an extended period, e.g., for greater than an hour). Often air bubbles may be trapped in a membrane, interfering with observation and study of cells held in the vessel. Further, certain materials added may be adsorbed or otherwise retained within a porous membrane.

In embodiments of a current damper of the present invention, a damping component is substantially an inner vessel of a solid-walled material that divides the vessel into a first volume and a second volume (as discussed above), where material added to the second volume passes to the first volume through at least one opening through the solid-wall of the damping component, such as current damper 58 depicted in Figure 6. By openings is intended openings such as, but not limited to, breaches, cracks, fenestrations, gaps, holes and perforations.

The size of each individual opening is generally large (relative to the cellular scale), generally having a cross section of at least about 0.4 x 10⁻³ mm², at least about 2.5 x 10⁻³ mm², at least about 10 x 10⁻³ mm², at least about 40 x 10⁻³ mm², at least about 0.25 mm², and even at least about 1 mm².

Although in embodiments a current damper has but one opening, preferably a given current damper has at least two openings, at least three openings and even at least six openings.

Although in embodiments of the present invention the openings are directed in any direction, including directed towards the bottom surface of the vessel, preferably the openings are not directed towards the bottom surface of the vessel, for example at least partially towards the wall of the vessel, towards the rim of the vessel or towards the opening of the vessel (*e.g,* current damper 58 depicted in Figure 6).

To ensure that all material added to a first volume passes into the second volume, it is preferable that the at least one openings are substatially disposed at a lowest point of the damping component.

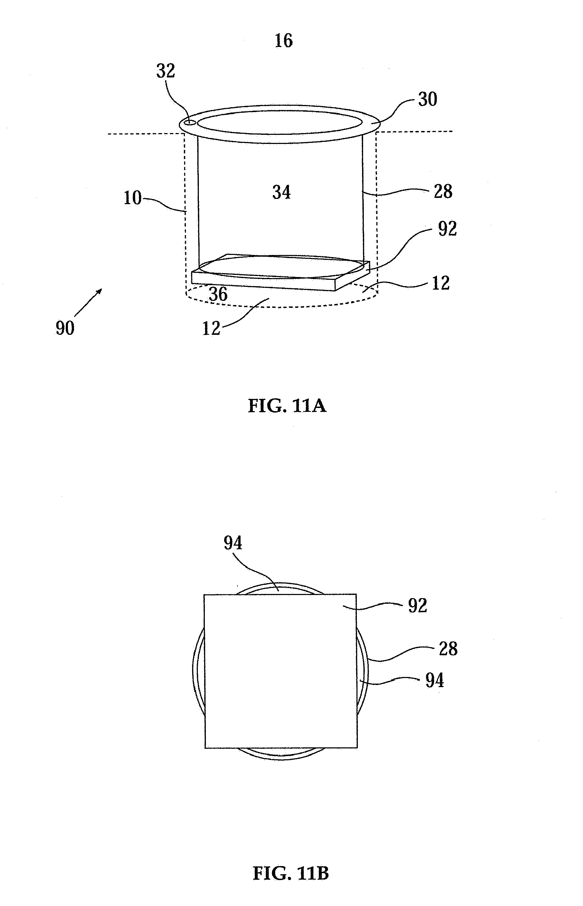

In Figures 11A and 11B is depicted a current damper 90 of the present invention: in Figure 11A in perspective disposed inside a vessel 10, depicted in phantom and in Figure 11B from the bottom. Current damper 90 is substantially similar to current damper 24 depicted in Figures 2A-2C with a significant difference that current damper 90 is provided with a solid non-porous bottom cover 92 fixedly attached to the bottom rim of circular wall 28. The dimensions of bottom cover 92 and circular wall 28 are such that there exist four gaps 94 at the bottom of current damper 90 that are not covered by bottom cover 92, see Figure 11B. Gaps 94 constitute openings directed towards bottom surface 12 of vessel 10 in the damping component of current damper 90, substantially wall 28 together with bottom cover 92.

For use, a physiological solution including cells is placed inside vessel 10 and the cells allowed to settle on bottom surface 12. The damping component of current damper 90 consisting of circular wall 28 and bottom cover 92 is placed inside vessel 10 so that flange 30 (constituting the retaining component of current damper 90) engages the top surface in the proximity of the rim of a vessel 10 as discussed above. As current damper 90 settles into vessel 10, trapped air escapes through pressure equalizing notch 32. When current damper 90 is in place, vessel 10 is divided into two volumes: a second volume 34 substantially above bottom cover 92 and a first volume 36 substantially between bottom cover 92 and bottom surface 12. First volume 36 is in fluid communication with surroundings 16 substantially only through gaps 94 via second volume 34. Material placed in second volume 34 passes through gaps 94 into first volume 36 and particularly to bottom surface 12 where cells are resting. The addition of material 22 to second volume 34 causes currents, but as passage of material 22 is through gaps 94, currents caused by the addition of material 22 to vessel 10 are substantially isolated to second volume 34 and damped in first volume 36 so the cells are only a little, not or substantially not disturbed and stay substantially in location on bottom surface 12.

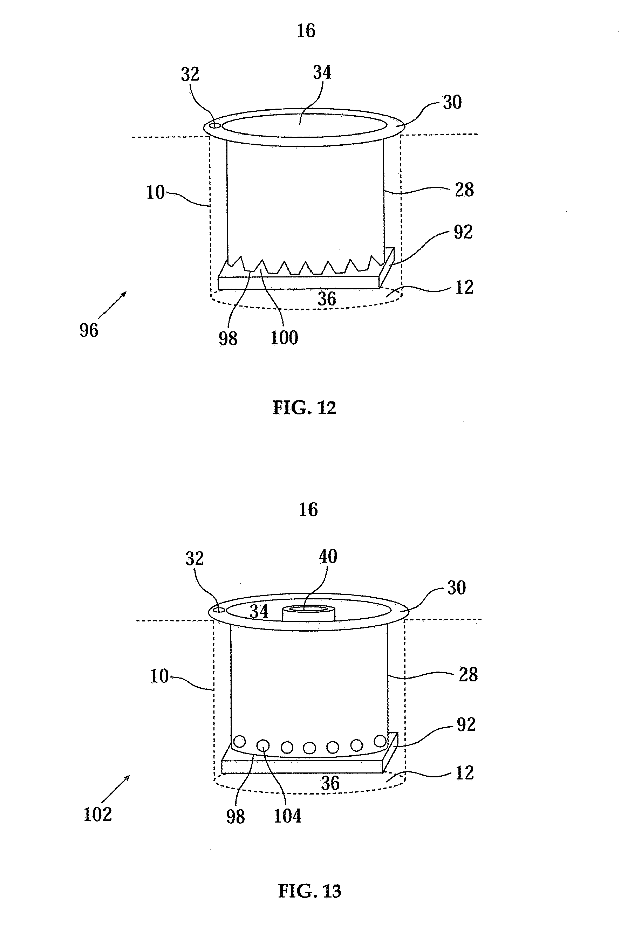

In Figure 12 is depicted a current damper 96 of the present invention in perspective disposed inside a vessel 10, depicted in phantom. Current damper 96 is substantially similar to current damper 90 depicted in Figures 11A-11B with a significant difference that solid non-porous bottom cover 92 of current damper 96 is of a size to contact the entire bottom rim 98 of circular wall 28. On bottom rim 98 are disposed a plurality (16 of which 7 are apparent) of triangular notches 100, formed by drawing a triangular file across bottom rim 98. Triangular notches 100 together with bottom cover 92 constitute openings directed towards the walls of vessel 10 in the damping component of current damper 96, substantially wall 28 together with bottom cover 92.

The use of current damper 96 is analogous to the use of current damper 90 as described hereinabove.

In Figure 13 is depicted a current damper 102 of the present invention in perspective disposed inside a vessel 10, depicted in phantom. Current damper 102 is substantially similar to current damper 96 depicted in Figure 12 with a significant difference that bottom rim 98 of circular wall 28 is smooth and featureless and makes uniform contact with bottom cover 92. However, through circular wall 28 are brought a plurality (12 of which 6 are apparent) of round holes 104 adjacent to bottom rim 98 of circular wall 28. Holes 104 constitute openings directed towards the walls of vessel 10 in the damping component of current damper 102, substantially wall 28 together with bottom cover 92. To bottom cover 92 is attached a tubular inner wall 40 that defines a direct and unimpeded passage from surroundings 16, through bottom cover 92 to first volume 36.

The use of current damper 102 is analogous to the use of current damper 90 (for damping of currents when adding a material) or of current damper 38 (for direct and unimpeded passage to first volume 36), as described hereinabove.

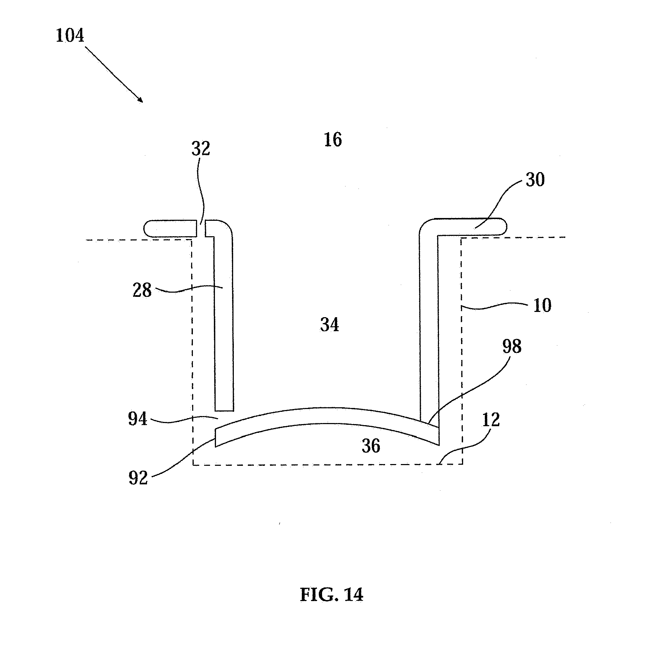

In Figure 14 is depicted a current damper 104 of the present invention in cross-section disposed inside a vessel 10. Current damper 104 is substantially similar to current damper 96 depicted in Figure 12 with a first significant difference that bottom rim 98 of circular wall 28 is crenellated so that openings 94 of current damper 104 are square or rectangular. A second significant difference is that bottom cover 92 is not flat, but rather lens shaped so that first volume 34 has a convex bottom. The crenellations of bottom rim 98 together with bottom cover 92 constitute openings 94 in the damping component of current damper 104 directed towards the walls of vessel 10. The convex bottom of first volume 34 ensures that openings 94 are at the lowest point of the damping component of current damper 104.

The use of current damper 104 is analogous to the use of current damper 90 as described hereinabove.

In embodiments of the present invention, construction of current dampers, such as current dampers 90, 96, 102 and 104 is relatively simple and involves fabrication of two separate components, a bottom cover 92 and a body including the other components of the incipient current damper from a suitable material, preferably a transparent material, especially for bottom cover 92. Suitable materials include but are not limited to glass, polycarbonate and polytetrafluoroethylene. Especially suitable are materials having an index of refraction similar to water, for example various fluorinated hydrocarbon polymers. By an index of refraction similar to the index of refraction of water is meant an index of refraction of not more than about 1.4, not more than about 1.38, not more than about 1.36, not more than about 1.35 and even not more than about 1.34, or substantially identical to that of water. Subsequently bottom cover 92 is associated, preferably fixedly associated, to a bottom rim 98 of a circular wall 28 of the body. Methods of attachment include but are not limited to clamping, welding and using an adhesive such as a light curable adhesive.

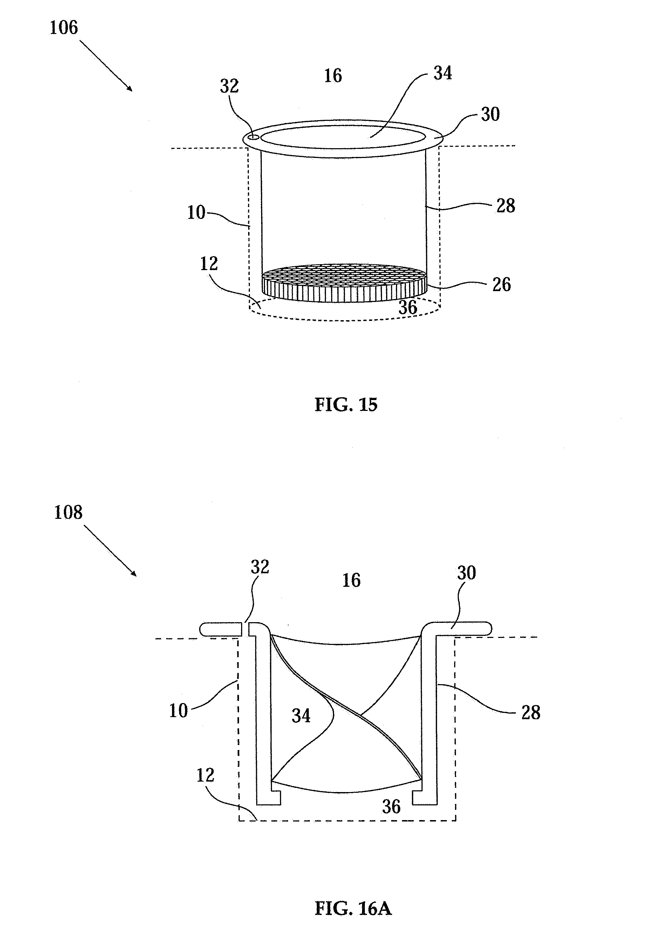

In Figure 15 is depicted a current damper 106 of the present invention in perspective in place inside a vessel 10, depicted in phantom. Current damper 106 is substantially similar to current damper 24 depicted in Figures 2A with a difference that a damping component 26 is substantially a bundle of borosilicate glass tubes fused together, each glass tube being 1 millimeter long and having a circular glass wall 10 micrometers thick and a 100 micrometers diameter bore (available, for example, as a preformed capillary array available, for example from Collimated Holes, Inc., Campbell, CA, USA).

The use of current damper 106 is substantially analogous to the use of current damper 24 as discussed hereinabove and depicted in Figures 2B and 2C. An advantage of current damper 106 relative to current damper 24 is that fluid flow through damping surface 26 comprising glass tubes is quicker than through a membrane such as a polycarbonate filter and that there is little chance of adsorption of materials onto the borrosilicate glass making up damping component 26 and that there is usually no need for soaking the glass tubes prior to use.

Embodiments of the present invention similar to current damper 106 having a greater flow rate therethrough (and thus, generally under usual conditions, a lesser current damping ability) may be provided, for example by using glass tubes shorter than 1mm (e.g., in embodiments no greater than 0.5 mm, or no greater than 0.2 mm) or having a greater bore (in embodiments greater than 100 micrometers, e.g. about 200 micrometers).

Embodiments of the present invention similar to current damper 106 having a slower flow rate therethrough (and thus, generally under usual conditions, a greater current damping ability) may be provided, for example by using glass tubes longer than 1mm (e.g., in embodiments no less than 1.5 mm, or no less than 2 mm) or having a smaller bore (in embodiments less than 100 micrometers, e.g. about 50 micrometers).

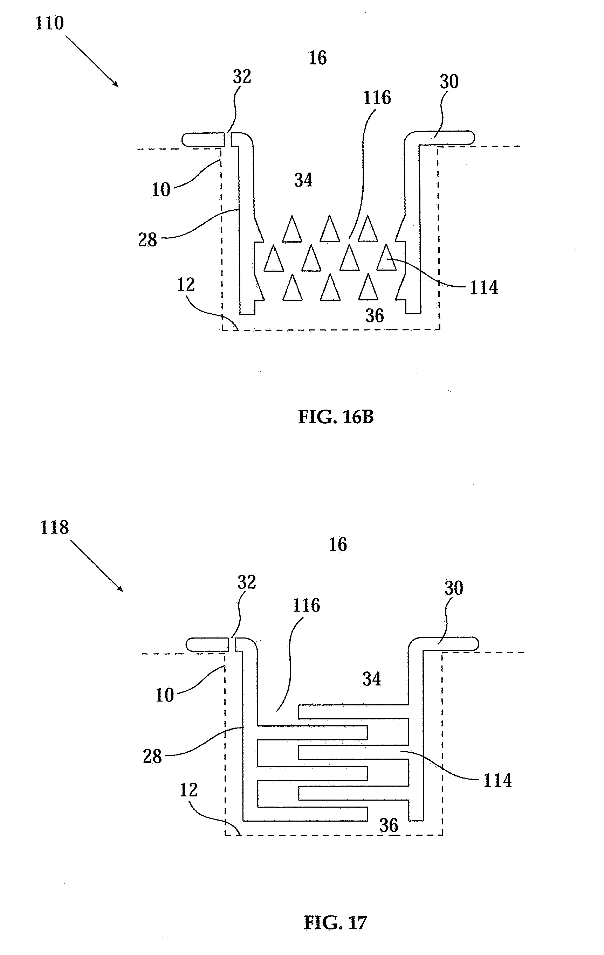

A disadvantage of some embodiments of the invention depicted above is the difficulty of adding larger sized materials, for example additional cells, into a vessel 10 where there are cells 20 already resting on a bottom surface 12 thereof. If additional cells are added through a second volume 34, the additional cells are caught on a damping surface 26. However, if additional cells are added directly to a first volume 36, e.g., through an upper opening 44 of a current damper 38 depicted in Figure 3, addition of the additional cells potentially disturbs cells 20 already resting on a bottom surface 12. In Figures 16A and 16B are depicted two embodiments of current dampers of the present invention, 108 and 110, that allow the addition of additional cells to a first volume 36 of a vessel 10 via a second volume 34 with little, if any, disturbing of cells 20 already resting on a bottom surface 12 of a vessel 10 in which the current dampers are placed.

In Figure 16A is depicted current damper 108 of the present invention in side cross section in place inside a vessel 10 with a bottom surface 12, depicted in phantom. Current damper 108 includes a circular wall 28, a flange 30 and a pressure equalizing notch 32 substantially as described above. Unlike embodiments discussed hereinabove, current damper 108 comprises a spiral wall 112 (depicted in perspective) as a non-planar and non-permeable damping component enclosed within circular wall 28. Spiral wall 112 defines a spiral fluid channel from surroundings 16 to bottom surface 12 of vessel 10.

The use of current damper 108 is analogous to the use of current dampers as described hereinabove. When a material, e.g. a liquid, is added to a vessel 10 provided with current damper 108, the material contacts spiral wall 112. Spiral wall 112 prevents the material from falling directly or splashing into vessel 10 but rather slows down the entry of the material, damping currents caused by the addition of the material and thus reducing the chance that cells resting on bottom surface 12 of vessel 10 will be displaced. Further, the outwards (centrifugal) component of motion imparted on the added material by interaction with spiral wall 112 provides for faster and relatively even mixing of the added material with liquid already inside vessel 10. Additionally, and in contrast to some of the embodiments discussed hereinabove, larger sized materials such as cells can also be added to a vessel 10 provided with current damper 108. For example, when cells are added, the cells start slowly rolling down the upper surface of spiral wall 112 until exiting from the bottom of current damper 108 and coming to rest on bottom surface 12 of vessel 10. It has been found that the outwards (centrifugal) component of motion imparted on cells by interaction with spiral wall 112 is sufficient to distribute the added cells over bottom surface 12 rather than concentrating these at any given location.

It is important to note that the extent of current damping and magnitude of the outwards component of motion imparted on materials exiting current damper 108 is in a large part dependent on the number of rotations and the angle of spiral wall 112. Thus, although spiral wall 112 of current damper 108 depicted in Figure 16A makes a half (180°) rotation, in embodiments a spiral wall makes at least about half a rotation, in embodiments at least about 3 rotations, at least about 6 rotations and even at least about 9 rotation. Although spiral wall 112 of current damper 108 is depicted as making an angle of about 45° (in proximity to circular wall 28), in embodiments a spiral wall makes an angle greater or less than about 45°. As lesser angles increase the degree of the damping effect, in embodiments, a spiral wall makes an angle of no greater than about 40°, no greater than about 30° and even no greater than about 25°.

In Figure 16B is depicted a current damper 110 of the present invention in side cross section in place inside a vessel 10 with a bottom surface 12, depicted in phantom. Current damper 110 includes a circular wall 28, a flange 30 and a pressure equalizing notch 32 substantially as described above. Unlike embodiments discussed hereinabove, the damping component of current damper 110 comprises a plurality (ten) of baffles 114, each baffle 114 being a glass rod with a triangular cross section spanning across the bore defined by circular wall 28 perpendicularly to circular wall 28. In current damper 110, baffles 114 are arranged in three rows, the baffles in each row being parallel with a gap 116 between, gap 116 being somewhat smaller than the widest portion of a baffle 114. Baffles 114 in a succeeding row are similarly arranged, but staggered so that below a gap 116 of a given row is positioned a baffle 114 of a succeeding row. In such a way, baffles 114 obstruct linear flow of fluids from surroundings 16 to bottom surface 12 of vessel 10.

Use of current damper 110 is substantially similar to the use of current damper 108 depicted in Figure 16A. When large materials such as additional cells are added, the additional cells roll down the sides of triangular baffles.

The extent of current damping in embodiments similar to current damper 110 is in a large part dependent on factors such as on the number of rows of baffles 114, the size of baffles 114, the size of gaps 116 and the shape of the cross section of baffles 114. In embodiments baffles have circular, diamond-shaped or other cross sections. A triangular cross section such as depicted in Figure 16B has a number of advantages: the point of a triangle and the relatively steep angle reduce the chance that particles such as added cells are stuck on a baffle and the turbulent flow produced in a fluid flowing past the bottom of a triangular cross section baffle encourages mixing of an added material and increases the damping effect of a current damper.

In Figure 17 is depicted a current damper 118 of the present invention in side cross section in place inside a vessel 10 with a bottom surface 12, depicted in phantom. Current damper 118 includes a circular wall 28, a flange 118 and a pressure equalizing notch 118 substantially as described above. Like current damper 110 depicted in Figure 16B, current damper 118 depicted in Figure 17 is provided with a plurality (six) of baffles 114. Baffles 114 of current damper 118 are substantially a series of planar shelves extending from the inner surface of circular wall 28 substantially perpendicular to circular wall 28, each such baffle being an incomplete disk so as to leave a gap 116 allowing fluid communication from the top to the bottom of a baffle 114 though a gap 116. Baffles 114 are arranged in such a way so that a given gap 116 is not across a gap 116 of a succeeding baffle 114. In such a way, baffles 114 obstruct linear flow of fluids from surroundings 16 to bottom surface 12 of vessel 10.

The extent of current damping in embodiments similar to current damper 118 is in a large part dependent on factors including the number of baffles 114 (a minimum of two), the size of baffles, the size of gaps 116, the shape of the cross section of baffles 114 and the distance between any two baffles 116.

The use of current damper 118 is substantially similar to the use of current dampers of the present invention discussed above.

It is important to note that embodiments of current dampers similar to current dampers 108, 110 and 118 may be less suitable than current dampers similar to current dampers 24 or 38 for use in embodiments where it is desired to dampen currents caused by placing a probe or detector into a fluid held inside a vessel 10 as the vertical height of obstruction caused by a spiral wall 112 or baffles 114 prevents such a probe or detector from closely approaching cells resting on a bottom surface 36 of a vessel 10.

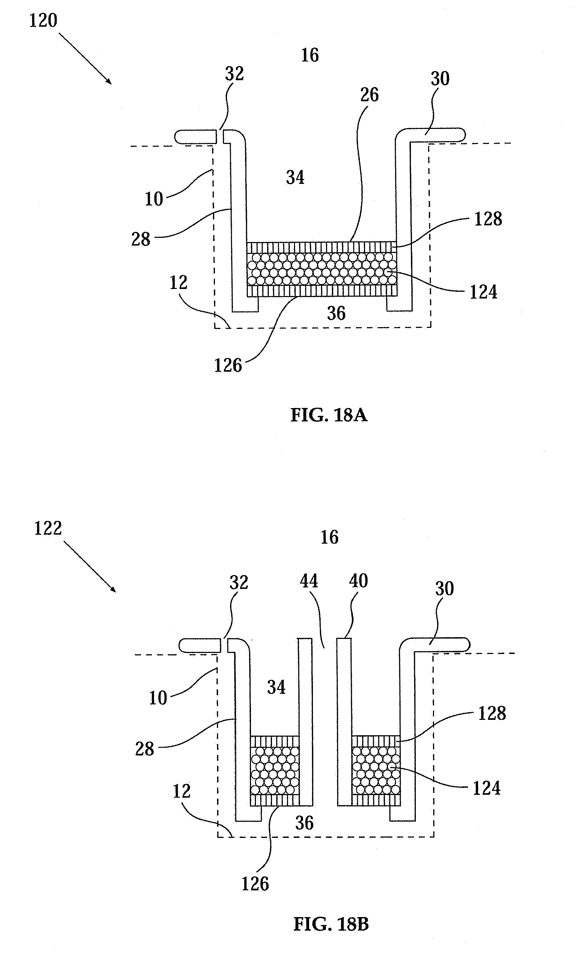

In embodiments of the present invention a damping component comprises a layer of small particles. A representative such current damper is current damper 120 depicted in Figure 18A or current damper 122 depicted in Figure 18B, both in side cross section where a layer 124 of small particles partially fills second volume 34.

Current damper 120 depicted in Figure 18A is substantially a current damper similar to current damper 24 depicted in Figure 2A provided with a 1mm layer 124 of glass beads having diameters of between 150 and 212 micrometers (e.g., G1145 from Sigma-Aldrich, St. Louis, MO, USA) so that fluid passages are defined through the layer that are no smaller than about 54 micrometers. To keep the particles making up layer 124 together and from falling into vessel 10, bottom retaining layer 126 is provided, comprising a thin layer (100 micrometers) of sintered glass having pores that are small enough to prevent passage of the particles therethrough, e.g., a sintered glass layer having pores of 40 - 100 micrometers (SIBATA Scientific Technology Ltd., Ikenohata, Taito-ku, Tokyo, Japan). Layer 124 is covered with a porous capping layer 128, similar to bottom retaining layer 126, in order to prevent loss of particles from current damper 120.

Current damper 122 depicted in Figure 18B is substantially a current damper such as current damper 38 depicted in Figure 3 provided with a 2mm layer 124 of 100 micrometer diameter glass beads (e.g., G4649 from Sigma-Aldrich, St. Louis, MO, USA) so that fluid passages are defined through the layer that are no smaller than about 36 micrometers. Similarly to current damper 120 depicted in Figure 18A, current damper 122 is provided with a porous capping layer 128 and a bottom retaining layer 126.

Use of current dampers such as 120 or 122 is substantially similar to the use of current dampers discussed hereinabove, and is clear to one skilled in the art upon perusal of the description herein.

Although the use of small particles as damping components in accordance with the teachings of the present invention is depicted with current dampers similar to current dampers 24 or 38, in embodiments such small particles are used together with other current dampers, e.g, current damper 90 of Figure 11A, current damper 96 of Figure 12 or current damper 102 of Figure 13.

Although in current dampers 120 and 122 layer 124 of small particles is the primary damping component responsible for the lion's share of current damping, in embodiments a layer of small particles is an additional, secondary or auxiliary damping component of a current damper. For example, in embodiments a porous capping layer and/or a bottom retaining layer are provided with smaller pores to increase the relative current damping by these components.

Small particles used as damping components in accordance with the teachings of the present invention are preferably non-absorbent, non-adsorbent, non-porous, inert and/or non-adhesive so as to allow a material added to a current damper including such particles to pass through chemically and biologically unaffected. Suitable small particles include sand, quartz powders, glass powders, glass beads, polystyrene beads and fluorinated hydrocarbon polymer particles such PTFE or fluorinated ethylene propylene. In embodiments, the particles are coated with a material that provides a desired degree of non-absorbance, non-adsorbance, non-porosity, inertness or adhesion prevention. That said, in embodiments the small particles have a hydrophilic or a hydrophobic surface. In embodiments, the small particles include a material that is released at a desired rate, for example, small particles configured to release nutrients at a controlled rate.

A current damper including a layer of small particles generally includes a component such as bottom retaining layer 126 configured to prevent passage of the particles making up a layer 124 therethrough. A current damper including a layer of small particles generally includes a component such as porous capping layer 126 configured to keep the particles making up a layer 124 in place. That said, in embodiments, a layer of loose particles is not held in place by a component such as a porous capping layer.

The flow rate of fluids through a layer of particles, such as a layer 124, and consequently the degree of current damping is dependent on a number of interacting and mutually dependent factors such as the thickness of the layer (thicker, greater damping), size of the particles (smaller particles, greater damping), shape of the particles (more irregular, greater damping), surface area of particles for volume of channels through the layer (greater surface area, greater damping) and extent of convolution of channels between the particles and through the layer (greater convolution, less linearity, greater damping).

The flow rate of fluids through a layer of particles, such as a layer 124, and consequently the degree of current damping is in part determined by the thickness of the layer. Typical thicknesses for useful implementations of the present invention are no less than about 0.5 mm, no less than about 1 mm, no less than about 2 mm and even no less than about 3 mm.

The flow rate of fluids through a layer of particles , such as a layer 124, is in part determined by the size of the channels between the particles and the ratio between the surface area of the particles for volume of channels through the layer. Generally the smaller the particles the smaller the channels between the particles and the greater the surface area of the particles. Further, as is known to one skilled in the art of packing, channel size may be reduced by using a mixture of particles of different sized so that smaller sized particles occupy channels defined by larger sized particles. Thus, in embodiments, the small particles making up a current damping layer are homogenous in size while in embodiments the small particles are heterogenous in size.

Small particles used as damping components in accordance with the teachings of the present invention are typically of any useful size. For example, in embodiments where it is desired to allow large materials to be added through a damping component (analogously to the discussed with regard to current damper 108 or 110), for example, additional cells, without fear that the large materials become trapped in the damping component, then a layer made up of relatively large particles are used although, in order to maintain sufficient damping effect such a layer may be relatively thick. For example, 100 micrometer particles define channels that are no smaller than about 36 micrometers, which are large enough for most cells to pass through and 200 micrometer particles define channels that are no smaller than about 73 micrometers. Thus, in embodiments, the particles making up a damping component are no less than about 100 micrometers in diameter, no less than about 200 micrometer and even no less than about 300 micrometers in diameter. In embodiments, it is not desired or not necessary (e.g., current damper 122 depicted in Figure 18B) to allow direct passage of large materials through second volume 34 and it is desired to provide a greater extent of current damping. Thus, in embodiments, the particles making up a damping component are no greater than about 100 micrometers in diameter, no greater than about 50 micrometer, no greater than about 20 micrometer and even no greater than about 10 micrometers in diameter.

As described above, in embodients the individual particles making up a damping component layer in accordance with the teachings of the present invention are loose and not physically connected so that the particles together constitute a powder necessitating the use of a capping layer such as 128. In embodiments the small particles are agglomerated, e.g. by pressure or sintering. Thus, in embodiments, the damping surface of a damping components is a unitary porous disk (e.g., for embodiments analogous to current damper 120) or torus (e.g., for embodiments analogous to current damper 122), such as a disk or torus of sintered glass known from the field of microfiltration that is configured to be held within a vessel such as a microwell and above the bottom surface of the microwell in accordance with the teachings of the present invention.

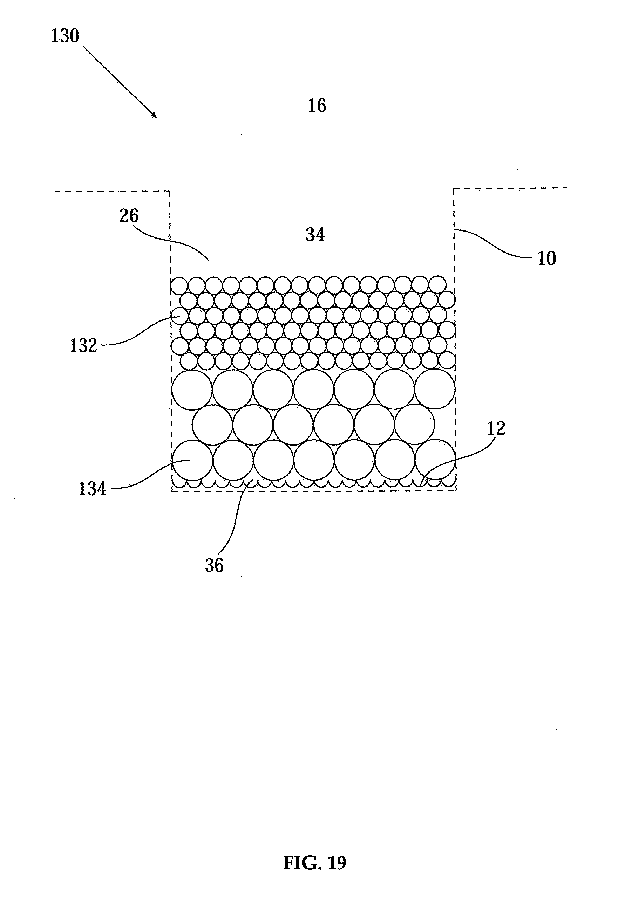

In Figure 19 is depicted an embodiment of the present invention where current damper 130, substantially only a current damping component, is seen in place inside a microwell 10 where on bottom surface 12 of microwell 10 are found circular picowells having a diameter of 20 micrometers as cell-localizing features, as described in PCT application PCT/IL2004/000661 published as WO 2005/007796 of the Inventor and hereinabove with reference to Figures 2B and 2C. Current damper 130 is made up of sintered inert glass beads in two layers, each layer including homogenously sized beads. Upper layer 132 consists of sintered glass beads having a diameter of 50 micrometers. The top of upper layer 132 defines in part a damping surface 26 of current damper 130. Lower layer 134 consists of sintered glass beads having a diameter of 200 micrometers. The size and shape of current damper 130 is such as to snugly slide into microwell 10 so that large glass beads making up lower layer 134 rest on bottom surface 12.

The use of current damper 130 is substantially as described above. After a solution including cells is placed inside microwell 10, the cells are allowed to settle into picowells on bottom surface 12. Current damper 130 is placed into microwell 10 and allowed to slide downwards until lower layer 134 rests on bottom surface 12. As the large beads are significantly larger than the picowells on bottom surface 12, only a few, if any, picowells are blocked and only a few, if any, cells are displaced or damaged. As is seen in Figure 19, first volume 36 is substantially the volume defined by the bottom the large beads making up lower layer 134 and bottom surface 12 of microwell 10. Second volume 34 is substantially the volume defined by microwell 10 above upper layer 132 of current damper 130. First volume 36 is in fluid communication with surroundings 16 substantially only through damping surface 26 via second volume 34.

The addition of a material such as a fluid to second volume 34 causes currents, but damping surface 26, upper layer 132 and lower layer 134 isolate the currents to second volume 34 so that cells are only a little, not or substantially not disturbed and stay substantially in location on bottom surface 12.

In embodiments, observation of cells held in a vessel provided with a current damper of the present invention is performed from the bottom of the vessel. That said, in embodiments, a current damper, or at least a damping component of a current damper, is transparent allowing light to pass through in order to allow adequate illumination of the vessel or even observation of cells through the current damper.

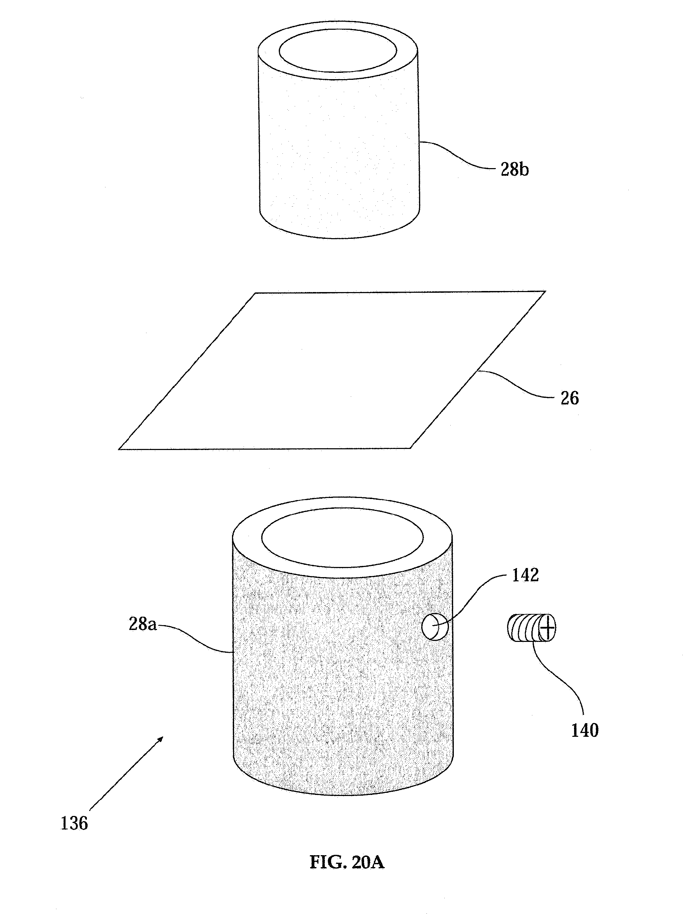

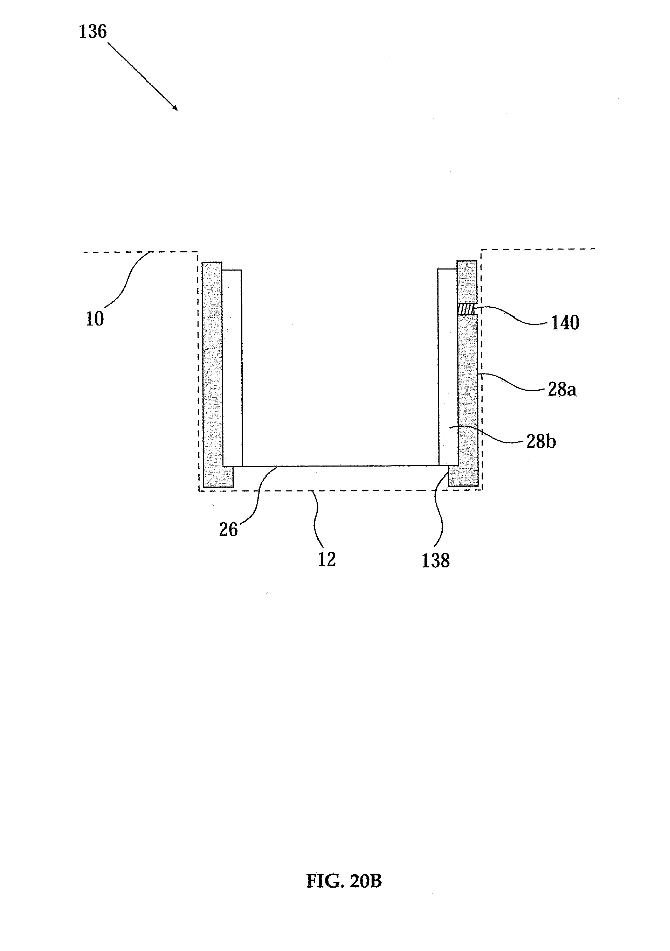

In Figures 20A and 20B a current damper 136 similar to current damper 24 of Figure 2A is depicted: in Figure 20A in exploded perspective and in Figure 20B in cross section disposed inside a vessel 10, a microwell of a standard 96-well plate. Current damper 136 includes a damping surface 26, an outer circular wall 28b, an inner circular wall 28b and a retaining screw 140.

Damping surface 26 is a 20 micron thick sheet of fluorinated ethylene propyleneTeflon® FEP with an index of refraction close to that of water from DuPont High Performance Films Circleville, OH, USA including 10 micron perforations.

Stainless steel inner circular wall 28b is configured to slidingly fit inside stainless steel outer circular wall 28a with a tolerance of about 20 microns. Outer circular wall 28a is configured to slidingly fit inside an appropriate vessel, for example a microwell 10 of a standard 96-well plate or of a 96-well plate provided with picowells as described in PCT patent application IL2004/000661 published as WO2005/007796 of the Applicant.

For assembly, damping surface 26 is stretched over inner circular wall 28b and outer circular wall 28a is slid over inner circular wall 28b until inner circular wall 28a contacts inwardly protruding lip 138 which is 200 micrometers high. Retaining screw 140 is screwed into hole 142 to press against inner circular wall 28b. In such a way, current damper 136 is held in an assembled state where damping surface 26 is held taut between outer circular wall 28a and inner circular wall 28b a distance of 200 micrometers above the bottom of outer circular wall 28a.

Use of current damper 136 is substantially similar to the use of current damper 24 depicted in Figure 2A with a few notable exceptions. For example, current damper 136 is configured to rest on bottom surface 12 of vessel 10 (constituting a retaining component to rest on the bottom of a surface of a vessel) and has no components that protrude above the upper portion of vessel 10. Damping surface 26 is accurately maintained at a desired distance above bottom surface 12 (200 micrometers) as a result of the fact that inner circular wall 28b rests on protruding lip 138. Further damping surface 26, when submerged in water, is transparent and invisible due to the fact that the index of refraction is close to that of water.

It is important to note that it is unexpected that a perforated fluorinated hydrocarbon polymer membrane such as damping surface 26 of current damper 136 made of Teflon® FEP is useful as a porous damping surface for implementing the teachings of the present invention. The hydrophobicity of such fluorinated hydrocarbons polymer is expected to prevent passage of water through the pores in the membrane. However, once both sides of damping surface are contacted with water, free (albeit slow) passage of water through the pores of the damping surface is observed. Further and also unexpectedly, once both sides of the perforated fluorinated hydrocarbon polymer membrane are contacted with water, no air is trapped in the membrane.

One of the important factors when using a current damper of the present invention is quick and efficient mixing, that is that the material added quickly passes from the second volume to the first volume and contacts the at least one cell found in the first volume and efficiently combines with the liquid in the first volume so that the at least one cell is in a substantially homogenous environment including the added material. However, as one purpose of a current damper of the present invention is to add material to a vessel in a manner that does not disturb cells resting on the bottom surface of the vessel, it is not practical to actively stir or mix the liquid or added material.

When the liquid in the vessel is water or an aqueous solution an unexpectedly efficient way to effect mixing is by adding a liquid material at temperature that is significantly lower than the temperature of the liquid in the vessel. Although it would be expected that the cold liquid material would sink quickly as a cohesive mass to the bottom surface of the vessel and that the greater rate of sinking would lead to eddy currents in the combined liquids in the vessel, this is not what happens. Rather, there is quick and efficient mixing with substantially no noticeable displacement of cells. Although not desiring to be limited to any one theory, apparently the greater density of the added liquid material coupled with the fact that the mass of cold liquid material is broken up into many streams when interacting with the damping component (especially when passing through a damping component that separates the vessel into a first volume and a second volume) leads to the observed efficient mixing. Further, significant eddy currents are not produced, apparently due to the damping effect of the static liquid in the vessel on the colder added material.

Mixing is increasingly efficient with an increasing difference in temperature between the liquid in the vessel and the added liquid material, with a noticeable increase in efficiency when the temperature difference is at least 10 °C, at least 20 °C and even at least 30 °C. Generally, the temperature of the liquid in the vessel is between about 34 °C and 40 °C and the temperature of added liquid material is less than about 20 °C, less than about 15 °C, less than about 10 °C and even less than about 5 °C.

### Methods of manufacture of a current damper of the present invention

In general, manufacture and assembly of a current damper of the present invention is well within the ability of one skilled in the art upon perusal of the description and figures herein using any suitable method with which one skilled in the art is well acquainted. Suitable methods include methods that employ one or more techniques including but not limited to casting, embossing, etching, free-form manufacture, injection-molding, microetching, micromachining, microplating, molding, spin coating, lithography or photo-lithography.

Suitable materials from which to manufacture components of a current damper include but are not limited to ceramics, epoxies, glasses, glass-ceramics, metals, plastics, polycarbonates, polydimethylsiloxane, polyethylenterephtalate glycol, polymers, polymethyl methacrylate, paraffins, polystyrene, polytetrafluoroethylene, polyurethanes, polyvinyl chloride, silicon, silicon oxide silicon rubbers.

In embodiments of the present invention it is preferred that at least part of a current damper of the present invention be transparent so as to allow illumination of cells resting at the bottom of a vessel in which the current damper is found through the current damper.

In embodiments of the present invention, it is preferred that a current damper be configured to allow observation of cells therethrough. For example, current damper 54 depicted in Figures 5A and 5B or current damper 58 depicted in Figure 6 are configured so as to present minimal interference to observation of cells resting on bottom surface 12 of vessel 10.

As noted above, in embodiments, components of a current damper of the present invention are fashioned from materials having an index of refraction similar to water.

In embodiments similar to current damper 24 depicted in Figure 2A a damping surface 26 is fashioned from a material having an index of refraction similar to water.

In embodiments similar to current damper 38 depicted in Figure 3 a damping surface 26 and/or ribs 42 are fashioned from a material having an index of refraction similar to water.

In embodiments similar to current damper 46 depicted in Figure 4 a damping surface 26 and/or ribs 52 are fashioned from a material having an index of refraction similar to water.

In embodiments similar to current damper 66 depicted in Figure 7, a current damper 70 depicted in Figure 8 or a current damper 74 depicted in Figure 9 a damping surface 26 and/or a frame 70, 72 or 76 respectively are fashioned from a material having an index of refraction similar to water.

In embodiments similar to current damper 90 depicted in Figure 11A, current damper 96 depicted in Figure 12, current damper 102 depicted in Figure 13 or current damper 104 depicted in Figure 14 a bottom cover 92 is fashioned from a material having an index of refraction similar to water.

In embodiments similar to current damper 106 depicted in Figure 15 the tubes making up damping surface 26 are fashioned from a material having an index of refraction similar to water (see U.S. Patent Application 11/062,479 published as US 2005/0232561).

In embodiments similar to current damper 110 depicted in Figure 16B or current damper 118 depicted in Figure 17 baffles 114 are fashioned from a material having an index of refraction similar to water.

In embodiments similar to current damper 120 depicted in Figure 18A, 122 depicted in Figure 18B or 130 depicted in Figure 13 small particles 124 and/or porous capping layer 128 and/or bottom retaining layer 126 are all fashioned from a material having an index of refraction similar to water.

By an index of refraction similar to the index of refraction of water is meant an index of refraction of not more than about 1.4, not more than about 1.38, not more than about 1.36, not more than about 1.35 and even not more than about 1.34, or substantially identical to that of water. Such components, once immersed in water, are virtually invisible and allow observation of cells therethrough. Exceptionally suitable such materials are fluorinated hydrocarbon polymers.

A fluorinated hydrocarbon polymer suitable for implementing the teachings of the present invention is fluorinated ethylene propylene (available, for example, as Teflon® FEP from DuPont High Performance Films Circleville, OH, USA having an index of refraction of 1.341-1.347 and a density of 2.15 g ml⁻¹) which is one of the most chemically inert plastics, has antistick properties, is not cytotoxic and is commercially available as a film from 12.5 micrometers and higher, as small particles of any desired size from submicronic diameters and as filaments (manufactured directly by extrusion or by slicing of sheets). A similar fluorinated ethylene propylene also suitable for fashioning components of a current damper of the present invention is Norton® FEP fluoropolymer film available from Saint-Gobain Performance Plastics, Wayne, NJ, USA having an index of refraction of 1.341-1.347 and a density of 2.12-2.17 g ml⁻¹.

An additional suitable fluorinated hydrocarbon polymer is the amorphous fluorocarbon polymer marketed under the tradename Cytop® (Asahi Glass Company, Tokyo, Japan) having an index of refraction of 1.34 and a density of 2.03 g ml⁻¹) and is commercially available as a film, as small particles of any desired size from submicronic diameters and as filaments (manufactured directly by extrusion or by slicing of sheets).

It is important to note that components of current dampers of the present invention, and especially damping components are preferably fashioned to allow passage of an added material therethrough without affecting the material. Thus, components of current dampers of the present invention and especially damping components are preferably non-absorbent, non-adsorbent, non-porous, inert and non-adhesive. One skilled in the art is familiar with suitable materials.

In embodiments of the present invention, a current damper includes a component made of a permeable membrane such as a microporous membrane. Suitable membranes for use as components of a device of the present invention include but are not limited to collagen, polyethersulfone, nitrocellulose mixed esters, polyamide (Nylon), polycarbonate, polyester, polyvinylidene fluoride, cellulose acetate and polytetrafluoroethylene. Suitable membranes are commercially available, for example, Magna^{™} membranes from Osmonics, Inc., Minnetonka, MN).

Membrane components of a current damper of the present invention are attached to other components of the current damper with the usual methods, for example using adhesives, welding or clamping of the membrane between two other components.

In a preferred embodiment, a damping component of the present invention is made of, at least in part, of a transparent material having an index of refraction that is close to that of water (e.g., less than about 1.4, less than about 1.38, or even less than 1.35), for example made of polytetrafluoroethylene. When such a material is used that part is effectively invisible, avoiding optical study of cells held in the vessel where the damping component is disposed. For example, when a damping surface 26 of a damping component such as 24 depicted in Figure 2A, 66 depicted in Figure 7, 70 depicted in Figure 8 or 74 depicted in Figure 9 is fashioned of a transparent material having an index of refraction close to that of water, such as a microporous polytetrafluoroethylene membrane, cells resting on bottom surface 12 may be observed and studied optically from above or below vessel 10.

### EXPERIMENTAL

A solution of approximately 18000 MOLT non-adherent cells in 50 microliter Dulbecco's phosphate buffered saline (PBS) were placed in each microwell of a standard 96-well plate (Nunc A/S, Roskilde, Denmark). After all the cells had settled (about 5 minutes) a current damper of the present invention substantially similar to current damper 94 depicted in Figure 10 was associated with the 96-well plate so that a damping component 86 was disposed within each one of the microwells of the 96-well plate. Each damping surface 26 was made of 3 micrometer microporous polycarbonate filter membrane (Osmonics, Inc, Minnetonka, MN, USA). The current damper was configured to suspend damping surfaces 26 200 micrometers above the microwell bottoms. The positions of cells resting on the bottom surface of a microwell were observed through the bottom surface of the microwell using an inverted microscope. Addition or removal of fluid into or from second volumes 34 did not cause any observable motion of the cells. 50 microliters of 4.8 micromolar Fluoresceine diacetate dye was added into second volume 34. The immediate emission of the characteristic Fluoresceine diacetate fluoresence from the cells indicated that the damping surface 26 did not cause a delay in delivery of the dye.

A current damper of the present invention substantially similar to current damper 94 depicted in Figure 10 was associated with standard 96-well plate (Nunc A/S, Roskilde, Denmark) so that a damping component 86 was disposed within each one of the microwells of the 96-well plate. Each damping surface 26 was made of 8 micrometer permeable collagen (Cellagen®, ICN Biomedicals Inc., Aurora, OH, USA). The current damper was configured to suspend damping surfaces 26 1 mm above the microwell bottoms. 50 microliters of a solution including approximately 20000 U937 non-adherent human promonocytes suspended in Dulbecco's phosphate buffered saline (PBS) were added through the tubular internal wall 40 of each damping component 86 and observed to form a single layer on the microwell bottom. The positions of cells resting on the microwell bottom surface were observed through the bottom surface using an inverted microscope. Addition or removal of fluid from second volume 34 did not cause any observable motion of the cells. Cells were removed from and added to the microwells using a pipette through a tubular internal wall 40.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art.

For example, although the embodiments depicted above are discussed with reference to a microwell of a 96-well plate with or without a picowell array on the bottom surface, the teachings of the present invention are applicable to any vessel used in the study of cells including but not limited to beakers, bottles, cups, flasks, plates, slides with recesses, tissue culture plates, vials, wells, microwells and Erlenmeyer flasks.

For example, although the embodiments depicted above include a single damping component disposed in a given vessel, the teachings of the present invention are also of a vessel having two or more damping components.

For example, although in the embodiments depicted above a vessel is divided into two volumes, a first volume including part of the bottom surface of the vessel and a second volume accessible from the surroundings and in fluid communication with the first volume through a damping component, the teachings of the present invention also include a vessel divided into more volumes, generally such volumes accessible from the surroundings and in fluid communication with the first volume through a damping component, analogously to the second volume.

For example, in the embodiments depicted above it is seen that various proportions of the area of an opening of a vessel 10 allow access to a respective first volume 36 and to a respective second volume 34 from surroundings 16. For example, in the embodiments depicted in Figure 2A or Figure 8 substantially all the area of the opening of vessel 10 allows access to second volume 34 from surroundings 16, while in the embodiment depicted in Figure 6 approximately 10% of the area of the opening of vessel 10 allows access to second volume 34 and 90% of the area allows access to first volume 36. In general, according to the teachings of the present invention any useful proportion of the area of an opening of a vessel 10 allows access to a respective first volume 36 and to a respective second volume 34 from surroundings 16. Generally at least *5%,* at least 10% and even at least 20% of the area of the opening of a vessel 10 allows access to a respective second volume 34. In embodiments of the present invention a first volume 36 is substantially not accessible from the surroundings, although in embodiments of the present invention at least 5%, at least 10% and even at least 20% of the area of the opening of a vessel 10 allows access to a respective first volume 36.

Accordingly, the present invention is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In case of conflict, the specification herein, including definitions, will control. Citation or identification of any reference in this application shall not be construed as an admission that such reference is available as prior art to the present invention.

## Claims

1. A current damper, comprising:
a) a layer including an upper surface, a lower surface and a width comprising a plurality of small particles including voids therebeween so as to define a plurality of non-linear fluid channels from said upper surface through said layer to said lower surface, rendering said layer permeable to fluids; and
b) a component limiting said width of said layer to a size configured to fit inside a vessel for the study of cells.

2. The current damper of claim 1, wherein said small particles comprise particles having a volume of no more than about 14x10⁶ micrometer³.

3. The current damper of claim 1, wherein said small particles comprise particles having a volume of no less than about 5.2x10⁻⁴ micrometer³.

4. The current damper of claim 1, said lower surface of said layer configured to rest on a bottom surface of a said vessel for the study of cells.

5. The current damper of claim 1, further comprising a bypass channel through said layer, defining a linear flow channel through said layer

6. A device for the study of cells, comprising:
(a) a vessel including a bottom surface and a wall having a bottom edge, a top edge defining a rim of said vessel, said rim surrounding an opening; and
(b) a current damper including a damping component substantially disposed within said vessel
wherein said damping component comprises a permeable layer of small particles including voids therebeween defining a plurality of non-linear fluid channels through said layer.

7. The device of claim 6, wherein substantially all fluid paths through said layer are said non-linear fluid channels.

8. The device of claim 6, wherein said small particles are loose.

9. The device of claim 6, wherein said small particles are mutually fixedly attached.

10. A device for the study of cells, comprising:
(a) a vessel including a bottom surface and a wall having a bottom edge, a top edge defining a rim of said vessel, said rim surrounding an opening; and
(b) a current damper including a damping component substantially disposed within said vessel
wherein said damping component is permeable to liquids, comprising a bundle of tubes.

11. A device for the study of cells, comprising:
(a) a vessel including a bottom surface and a wall having a bottom edge, a top edge defining a rim of said vessel, said rim surrounding an opening; and
(b) a current damper including a damping component substantially disposed within said vessel
wherein said damping component comprises a spiral wall defining a spiral fluid channel from the surroundings to said bottom surface of said vessel.

12. A device for the study of cells, comprising:
(a) a vessel including a bottom surface and a wall having a bottom edge, a top edge defining a rim of said vessel, said rim surrounding an opening; and
(b) a current damper including a damping component substantially disposed within said vessel
wherein said damping component comprises at least two baffles configured to obstruct linear flow of fluids past said at least two baffles from the surroundings to said bottom surface of said vessel.

13. A device for the study of cells, comprising:
(a) a vessel including a bottom surface and a wall having a bottom edge, a top edge defining a rim of said vessel, said rim surrounding an opening; and
(b) a current damper including a damping component substantially disposed within said vessel.
wherein said damping component substantially divides said vessel into at least two volumes, a first volume including at least part of said bottom surface and a second volume in fluid communication with the surroundings and with said first volume and wherein said damping component is substantially an inner vessel, substantially defining said second volume, provided with at least one opening allowing fluid communication between said second volume and said first volume therethrough wherein said at least one opening is directed towards said wall of said vessel.
